(19) **Europäisches Patentamt**
**European Patent Office**
**Office européen des brevets**

(11) **EP 1 600 775 B1**

(12) **EUROPÄISCHE PATENTSCHRIFT**

(45) Veröffentlichungstag und Bekanntmachung des Hinweises auf die Patenterteilung:
**01.10.2008 Patentblatt 2008/40**

(51) Int Cl.:
*G01N 33/579* (2006.01)     *G01N 33/92* (2006.01)
*G01N 33/53* (2006.01)

(21) Anmeldenummer: **05009273.3**

(22) Anmeldetag: **28.04.2005**

(54) **Verfahren zur Anti-Limulus-Faktoren-unabhängien Bestimmung der Lipopolysaccarid- und/ oder Lipid-A- und/oder Glukan-Reaktivität**

Method for the anti-limulus-factors independent determination of lipopolysaccharide, and/or Lipid A, and/or glucan reactivity

Méthode pour la détermination de la réactivité de lipopolysaccharide et/ou de lipide A et/ou de glucan, cette détermination étant indépendante de facteurs anti-limulus

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **26.05.2004 DE 102004025780**

(43) Veröffentlichungstag der Anmeldung:
**30.11.2005 Patentblatt 2005/48**

(73) Patentinhaber: **Stief, Thomas, Dr.**
**35415 Pohlheim (DE)**

(72) Erfinder: **Stief, Thomas, Dr.**
**35415 Pohlheim (DE)**

(74) Vertreter: **Ackermann, Joachim**
**Postfach 11 13 26**
**60048 Frankfurt am Main (DE)**

(56) Entgegenhaltungen:
**DE-A1- 19 549 117      US-A- 3 954 663**
**US-A- 4 495 294**

- **TSUJI K ET AL: "RECOVERY OF ENDOTOXIN FROM HUMAN PLASMA BY ACID OXIDATIVE TREATMENTS AS MONITORED BY AN AUTOMATED MICROTITER PLATE-CHROMOGENIC SUBSTRATE LIMULUS AMEBOCYTE LYSATE (LAL) ASSAY" PROGRESS IN CLINICAL AND BIOLOGICAL RESEARCH, NEW YORK, NY, US, Bd. 231, 1987, Seiten 443-457, XP008054515 ISSN: 0361-7742**
- **HURLEY JAMES C: "Endotoxemia: Methods of detection and clinical correlates" CLINICAL MICROBIOLOGY REVIEWS, WASHINGTON, DC, US, Bd. 8, Nr. 2, 1995, Seiten 268-292, XP002305904 ISSN: 0893-8512**
- **OBAYASHI T: "ADDITION OF PERCHLORIC ACID TO BLOOD SAMPLES FOR COLORIMETRIC LIMULUS TEST USING CHROMOGENIC SUBSTRATE: COMPARISON WITH CONVENTIONAL PROCEDURES AND CLINICAL APPLICATIONS" JOURNAL OF LABORATORY AND CLINICAL MEDICINE, ST. LOUIS, MO, US, Bd. 104, Nr. 3, 1984, Seiten 321-330, XP008054518 ISSN: 0022-2143**
- **OCHIAI MASAKI ET AL: "A limulus amoebocyte lysate activating activity (LAL activity) that lacks biological activities of endotoxin found in biological products" MICROBIOLOGY AND IMMUNOLOGY, Bd. 46, Nr. 8, 2002, Seiten 527-533, XP002354593 ISSN: 0385-5600**

**Beschreibung**

Die vorliegende Erfindung betrifft einen verbesserten Test zur Bestimmung der Lipopolysaccharid- und/oder Lipid-A- und/oder Glukan-Reaktivität in Körperflüssigkeiten.

Unter Reaktivität wird im Rahmen dieser Beschreibung die Aktivität von Lipopolysaccharid und/oder Lipid-A und/oder Glukan, insbesondere beta-1,3-D-Glukan in einer biologischen Flüssigkeit, wie beispielsweise Plasma, verstanden. Reaktives Lipopolysaccharid und/oder Lipid-A und/oder Glukan ist pathophysiologisch und/oder pharmakologisch-toxikologisch aktives und/oder freies Lipopolysaccharid und/oder Lipid-A und/oder Glukan.

Infektionen mit gramnegativen Bakterien (wie *Escherichia coli*) oder mit Pilzen (wie *Candida albicans*) stellen bereits seit langem ein großes medizinisches Problem dar. Die Keime können in die Blutbahn oder die Meningen eindringen und dort zu lebensbedrohlichen Zuständen, wie Sepsis oder Meningitis, führen. Daher sollte die Diagnose dieser medizinisch so relevanten Erreger möglichst frühzeitig gestellt werden.

Mikrobiologische Verfahren der Anzüchtung dieser Keime sind jedoch sehr langwierig. In vielen Fällen kann die Diagnose erst nach Tagen oder nach Wochen gestellt werden. In wenigen Stunden kann sich allerdings das Krankheitsbild des Patienten derart verschlechtern, so daß er im schlimmsten Fall an diesem Infekt stirbt.

Es hat daher nicht an Versuchen gefehlt, den Zeitraum für das Stellen einer Diagnose zu verkürzen. Einer dieser Ansätze ist der Nachweis von Lipopolysacchariden (nachstehend auch "LPS" genannt), von Lipid-A oder von Glukanen mit dem sogenannten Limulus-Assay. LPS, Lipid-A und Glukane sind für den Patienten gefährlich, da deren Anwesenheit im Blut zu einer pathologischen disseminierten intravaskulären Gerinnung (DIC) führen können.

Die Zellwand der gramnegativen Bakterien besteht zum Teil aus LPS (früher Endotoxin genannt), welches sich zusammensetzt aus einem harmlosen Polysaccharid-Teil und einem für viele Zellen gefährlichen Lipid-A-Teil.

Die LPS- und/oder Lipid-A- und/oder Glukan-Reaktivität ist von hoher pathophysiologischer Bedeutung: aktives LPS und/oder freies LPS und/oder aktives und/oder freies Lipid A und/oder aktives und/oder freies Glukan, reagiert zum Beispiel sehr intensiv mit den Monozyten des Blutes oder dem Endothel. Die Monozyten können dabei stark aktiviert oder zerstört werden, und Zellinneres gerät in Kontakt mit Plasma und Thrombozyten.

Da Monozyten DNA und sogenannten Tissue-Factor enthalten, wird die Gerinnung bei Sepsis sofort aktiviert. Dabei kommt es folglich zu lebensbedrohlich-gesteigerter disseminierter intravaskulärer Gerinnung mit z. B. multipler Thrombosierung kleiner Gefäße, was zum Multiorganversagen führen kann.

Mit der vorliegenden Erfindung wird ein Verfahren bereitgestellt, welches die pathophysiologische Interaktion, d.h. die Reaktivität, von LPS bzw. Lipid-A bzw. Glukan mit empfindlichen Zellen imitiert, wobei die Zellen und/oder Zellmembranen durch die Limulusfaktoren in vitro ersetzt werden. Ein solches Verfahren, welches innerhalb von Minuten LPS, Lipid-A oder Glukane, insbesondere reaktives und/oder freies LPS und/oder reaktives und/oder freies Lipid-A und/oder reaktive und/oder freie Glukane, sensitiv und spezifisch nachweist, ist demzufolge von großer klinischer Bedeutung.

Nach dem Stand der Technik wird LPS mit dem Limulus-Assay nachgewiesen [Hurley J.C. Endotoxemia: methods of detection and clinical correlates. Clinical Microbiology Reviews 1995; 8: 268-292]. Das Prinzip dieses Testes besteht darin, daß LPS - aber auch Glukane - das Coagulogen/Coagulin System der Horseshoe-Krabbe (Pfeilschwanzkrebs; Limulus polyphemus), genannt das Limulus-System, aktivieren.

Das Limulus-System für LPS oder Lipid A besteht im wesentlichen aus folgenden Faktoren: Faktor C, Faktor B, Faktor A und Coagulogen.

LPS oder Lipid-A aktiviert Faktor C, aktivierter Faktor C aktiviert Faktor B, aktivierter Faktor B aktiviert Faktor A. Aktivierter Faktor A wandelt lösliches Coagulogen in unlösliches Coagulin um. Beim Limulus-System für Glukane, die Pilzbestandteile sind, gibt es noch einen Faktor G, der durch Glukane, insbesondere beta-1,3-D-Glukan zu einer Serinprotease aktiviert wird, wodurch aktivierter Faktor A gebildet wird.

Das Limulus-System ist ein Abwehrsystem des Pfeilschwanzkrebses gegen eindringende pathologische Keime und stellt ein primitives System der angeborenen Immunität dar.

Beim Limulus-Assay wirkt LPS und/oder Lipid-A und/oder Glukane der Probe auf Limulus Amöbocyten-Faktoren, im folgenden Limulus-Faktoren genannt, ein und führt zu Veränderungen, die auf unterschiedliche Arten nachgewiesen werden können (Hurley J., Clin. Microbiol. Rev. 8(2), 268-292, 1995, Ochiai et al., Microbiol. Immunol. 46, 527-533, 2002). Diese Limulus-Faktoren werden nach dem Stand der Technik aus dem Lysat von Limulus-Amebozyten gewonnen (US 3,954,663), woher die Abkürzung LAL stammt.

Beispiele für Nachweisverfahren sind der LAL-Gelierungstest, der Coagulogenbasierte LAL Assay und der chromogene LÄL Assay (vergl. Hurley J.C., Clinical Microbiology Reviews 1995; 8: 272).

Beim chromogenen LAL Assay oder chromogenen Limulustest wird die Reaktion des aktivierten Faktors A auf das Coagulogen durch Ersatz des Coagulogens durch ein chromogenes Substrat imitiert, z. B. S-2834® (Isoleucyl-Glutamyl-Glycyl-Arginylparanitro-Anilid = Ile-Glu-Gly-Arg-pNA) oder S-2423® (CH3-CO-Ile-Glu-Gly-Arg-pNA) (Chromogenix, Mölndal, Schweden).

Der chromogene Limulustest ist kommerziell erhältlich, beispielsweise unter den Handelsnamen Limulus Amebocyte Lysate Endochrome® (Charles River Endosafe; Charleston, USA), Coatest® Plasma Endotoxin oder Coamatic® Endotoxin (Chromogenix, Mölndal, Schweden) oder Pyrochrome® (Cape Code; Falmouth, USA).

Der chromogene Limulus-Test kann in der sogenannten Endpunkt-Hyperbolic-Rate-Assay-Kinetik durchgeführt werden (bei 37˚C, Ikubationszeit 41 min für Plasma, Produktinformation Pyrochrome®, Haemochrom, Essen, Deutschland), d.h. chromogenes Substrat und Limulus-Faktoren C, B, A werden gemeinsam der auf Endotoxin zu testenden Probe zugesetzt. So erfolgen die Aktivierung der Faktoren C, B, A und die Substratspaltung gleichzeitig. Je länger der Test vor Zusatz des Stop-Reagenz läuft, desto mehr aktivierter Faktor A wird generiert und desto mehr chromogenes Substrat wird gespalten.

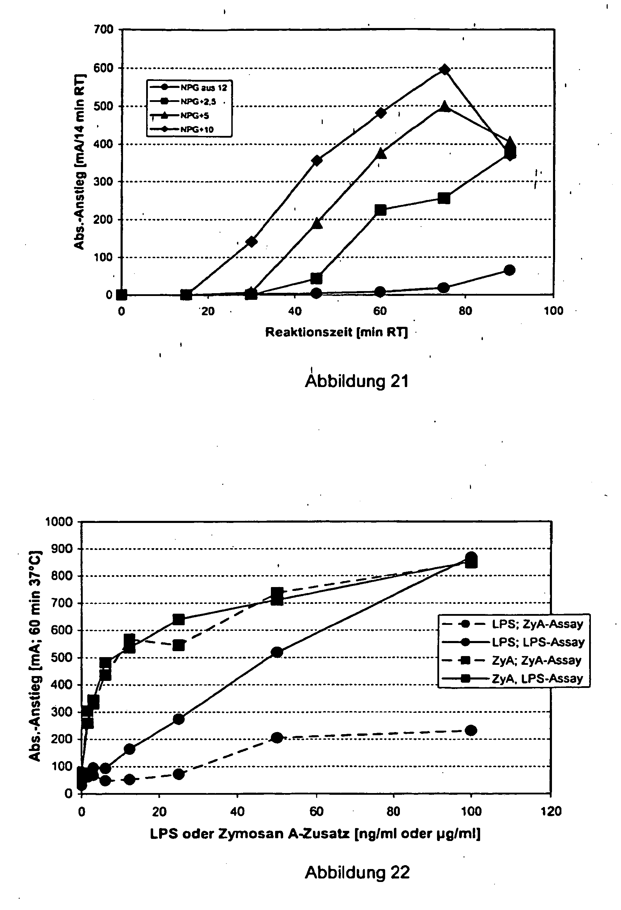

Alternativ kann das chromogene Substrat auch erst ein Zeitintervall nach dem Zusatz der Faktoren C, B, A zugesetzt werden. Dieses hat den Nachteil, daß während der Einwirkung des Endotoxins auf die Faktoren C, B und A vermehrt autokatalytische und/oder autodestruktive Interaktionen stattfinden (s. Abb. 21). Das chromogene Substrat (wie im Hyperbolic-Rate-Assay gegeben) inhibiert teilweise diese Interaktionen.

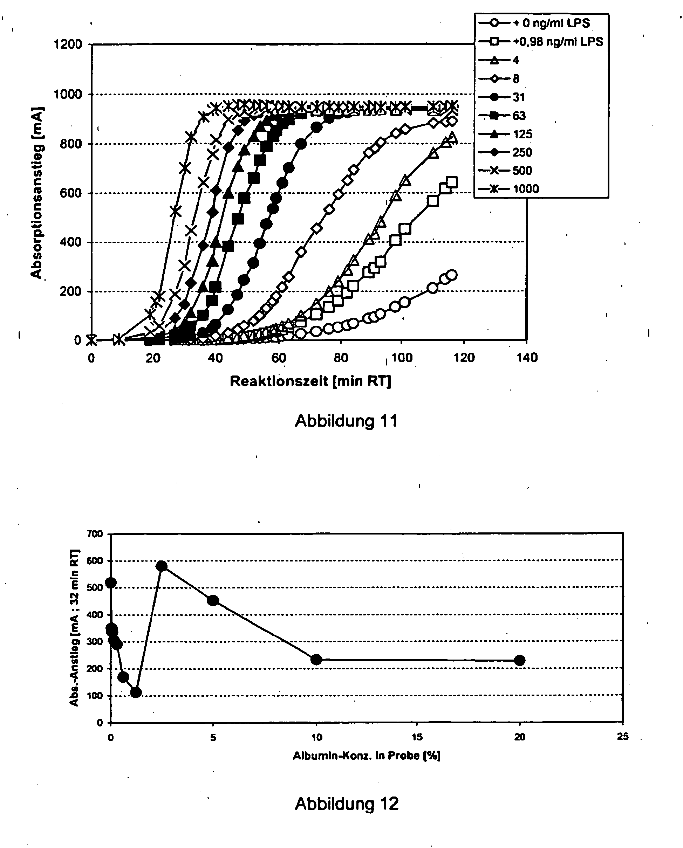

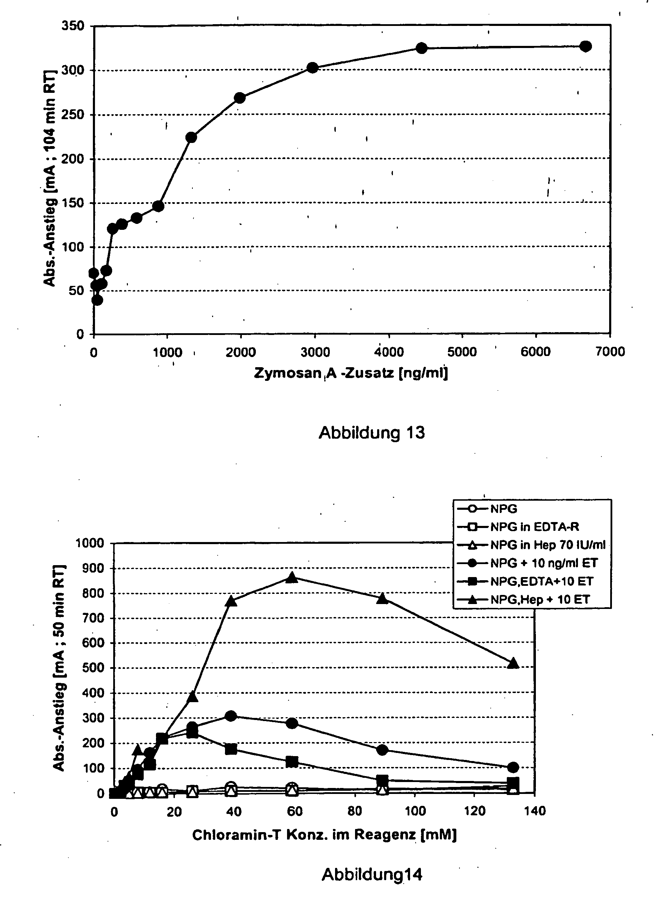

Das Haupt-Problem mit dem Limulus-Test ist, daß in der Probe enthaltene Anti-Limulusfaktoren, wie beispielsweise Proteine, insbesondere Serinprotease-Inhibitoren, wie Antithrombin III und/oder Heparin Cofaktor II und/oder alpha2-Antiplasmin, die Aktivierung des Limulus-Systems stören. Dies bedeutet, daß das Ergebnis des Limulus-Tests u.a. abhängt von dem Gehalt an Anti-Limulusfaktoren, wie beispielsweise Proteinen, insbesondere Serinprotease-Inhibitoren wie Antithrombin III und/oder Heparin Cofaktor II und/oder alpha2-Antiplasmin, in der Probe (s. Abb. 12,14).

Diese Interferenz wird nach dem Stand der Technik beseitigt, indem die Probe 10-fach verdünnt wird und die verdünnte Probe vor dem eigentlichen Test erhitzt wird, beispielsweise 15-20 min bei 75˚C. Alternativ kann die Probe auch stark angesäuert werden, um die Anti-Limulus-Faktoren zu inaktivieren (siehe Obayashi, T. et al. Clin. Chim. Acta 149, 55-65, 1985; J. Lab. Clin Med. 4, 321-330, 1984; US 4,495,294 Tsuji et al., Prog. Clin. Biol. Res., 231, 443-457, 1987) Dieses Prozedere ist jedoch problematisch, da von physiologischen Inhibitoren gebundenes und damit inaktives LPS und/oder Lipid-A und/oder Glukan wieder aus der Bindung freigesetzt werden kann, d.h. die Methode mißt nicht hauptsächlich reaktives und/oder aktives und/oder freies, sondern insbesondere auch gebundenes LPS oder Lipid-A bzw. Glukan, was letztlich dazu führt, daß die Methode nicht routinetauglich ist. Inhibiertes LPS bzw. Lipid-A bzw. Glukan hat untergeordnete pathophysiologische Bedeutung. Außerdem werden bei Erhitzung das in der Probe vorhandenen aktive und/oder freie LPS oder Lipid A nahezu vollständig inaktiviert. Die kommerziell erhältlichen Limulus-Tests haben unter anderem aufgrund dieser Probleme bislang keinen Eingang in die Klinikroutine gefunden.

Beispielsweise gibt es plasmatische Antikörper und andere Binde-Proteine, wie das Lipopolysaccharid-Binding-Protein (LBP), die LPS und/oder Lipid-A und/oder Glukane neutralisieren. Dieses gebundene und dadurch neutralisierte LPS und/oder Lipid-A und/oder Glukan ist jedoch von untergeordneter klinischer Bedeutung. Von pathophysiologischer Bedeutung ist LPS, Lipid-A oder Glukan (wie beispielsweise die Candida-Substanz Zymosan A), welches nicht-neutralisiert und/oder frei ist und somit frei mit empfindlichen Zellen wie beispielsweise den Monozyten des Blutes reagieren kann, sogenanntes reaktives LPS bzw. Lipid-A bzw. Glukan. Ein Freisetzen dieses neutralisierten LPS und/oder Lipid-A und/oder Glukan z.B. durch Hitzebehandlung würde das Testergebnis somit verfälschen und nur eine äußerst bedingte Aussage über die Konzentration des nicht-neutralisierten LPS und/oder Lipid-A und/oder Glukan gestatten.

Weiterhin ist ein Verfahren zur Bestimmung der Aktivität von Limulus-Endotoxinaktivierbaren Proteasen bekannt (DE 195 49 117).

Ausgehend von diesem Stand der Technik lag der vorliegenden Erfindung die Aufgabe zugrunde, ein einfaches Verfahren bereitzustellen, mit denen der Gehalt an aktivem LPS und/oder aktivem Lipid-A und/oder aktivem Glukan einer Probe, d.h. die LPS-Reaktivität und/oder Lipid-A-Reaktivität und/oder Glukan-Reaktivität, rasch bestimmt werden kann.

Eine weitere Aufgabe der vorliegenden Erfindung war die Bereitstellung eines Verfahrens, mit denen zwischen aktivem LPS bzw. aktivem Lipid-A und aktivem Glukan einer Probe differenziert werden kann.

Eine weitere Aufgabe der vorliegenden Erfindung war die Bereitstellung eines Verfahrens, mit denen das in einer Probe vorhandene aktive und/oder freie LPS und/oder Lipid-A und/oder Glukan ermittelt werden kann und die auf das in einer Probe vorhandene neutralisierte LPS und/oder Lipid-A und/oder Glukan nicht ansprechen.

Noch eine weitere Aufgabe der vorliegenden Erfindung war die Bereitstellung eines Verfahrens, deren Einsatz mit einer linearen Kinetik erfolgt und/oder die routinetauglich sind.

Noch eine weitere Aufgabe der vorliegenden Erfindung war die Bereitstellung eines Verfahrens, bei deren Durchführung auf die vorhergehende Verdünnung und/oder Erhitzung der Probe zur Bestimmung des LPS und/oder Lipid-A und/oder Glukan verzichtet werden kann.

Überraschenderweise hat sich gezeigt, daß durch Zusatz eines geeigneten Oxidationsmittels, gegebenenfalls in Verbindung mit einem Detergenz, wie beispielsweise Triton-X100®, vorzugsweise in einer finalen Konzentration bis zu 0,1%, und/oder einem Lösungsvermittler, wie beispielsweise Polyethylenglykol, vorzugsweise in einer finalen Konzentration bis zu 10 %, die Interferenz der in der Matrix der Probe (= Analyt-Umgebung) vorhandenen Störfaktoren auf das Limulus-System ausgeschaltet werden kann.

Es ist zwar aus der EP-A-297,597 bekannt, daß durch die oxidative Behandlung von biologischen Flüssigkeiten die Aktivität von Serinproteasen oder Serinproteaseinhibitoren bestimmt werden kann, doch gibt dieses Dokument keinerlei Hinweis auf den Einsatz von Oxidationsmitteln beim Limulus-Assay. Außerdem hat sich erfindungsgemäß herausgestellt, daß beim Einsatz mit dem Limulus-System erheblich höhere Oxidans-Dosen erforderlich sind, die beispielsweise mehr als 5-10fach über der Dosis liegen, welche für die oxidative Inaktivierung von oxidansempfindlichen Serinproteaseinhibitoren erforderlich sind.

Die vorliegende Erfindung betrifft ein Verfahren zur Bestimmung des Gehaltes an aktivem und/oder freiem Lipopolysaccharid und/oder Lipid-A und/oder Glukan in biologischen Flüssigkeiten bei einem pH-Wert zwischen 6 und 10 gemäß Anspruch 1 umfassend

a) die Behandlung der Probe mit mindestens einem Oxidationsmittel, aus der Gruppe der Singulett-Sauerstoff-Erzeuger,
b) den Zusatz von mindestens einem Limulus Amöbocyten - Faktor zur oxidierten Probe, und
c) die Bestimmung der Aktivierung mindestens eines der Limulus Amöbocyten - Faktoren.

Erfindungsgemäß eingesetzte Oxidationsmittel können beliebiger Natur sein, soweit dadurch die Oxidation von Anti-Limulus Faktoren der Probe, insbesondere Antithrombin III und/oder Heparin Cofaktor II und/oder alpha2-Antiplasmin bewirkt werden kann.

Beispiele für Oxidationsmittel sind Singulett-Sauerstoff ($^1O_2$)-Erzeuger wie Chloramine oder Salze der HOCl, insbesondere Chloramin-T® (N-Chlor-Toluol-Sulfonamid) oder Chloramin-B® (N-Chlor-Benzol-Sulfonamid).

Die erfindungsgemäß eingesetzten Oxidationsmittel kommen in vergleichsweise hohen Dosen zum Einsatz, um die Anti-Limulus Faktoren zu inaktivieren ohne jedoch die Reaktivität von LPS und/oder Glukan zu verändern, insbesondere gilt es zu vermeiden, in der Probe vorliegendes inaktives und/oder gebundenes LPS und/oder Glukan im Testverfahren zu reaktivieren und/oder aus der Bindung freizusetzen und aktives LPS oder Lipid A und/oder Glukan nicht zu inaktivieren.

Typischerweise sind Oxidans-Dosen erforderlich, die mehr als 5-10fach über den Dosen liegen, welche für die oxidative Inaktivierung von oxidansempfindlichen Serinproteaseinhibitoren, beispielsweise Antithrombin III oder $\alpha$2-Antiplasmin im Plasma erforderlich sind.

Die Konzentration des Oxidationsmittels, beispielsweise von Chloramin-T®, im Oxidansreagenz beträgt üblicherweise 15 - 200 mmol/l, insbesondere 30-120 mmol/l, besonders bevorzugt 50-70 mmol/l.

Das Oxidationsmittel wird der Probe typischerweise in einer Menge von 15-200 $\mu$mol, insbesondere 30-120 $\mu$mol, besonders bevorzugt 50-70 $\mu$mol pro ml Probenflüssigkeit, insbesondere Plasma, zugesetzt. Das Oxidans kann gegebenenfalls in physiologischer NaCl-Lösung und/oder in einem Puffer vorliegen, insbesondere PBS (pH 7.4), NaHCO$_3$ (pH 8 - 9), oder Citrat (pH 7 - 9, bevorzugt 7.4 - 8.4), bevorzugt Citrat, insbesondere bevorzugt Natrium-Citrat, in einer Konzentration von 10 - 200 mM, insbesondere 20 - 100 mM, besonders bevorzugt 50 mM, wenn das Verhältnis Probe : Oxidansreagenz 1:1 beträgt.

Niedrigere Dosen beseitigen in der Regel nicht den störenden Einfluß der Plasmamatrix auf das Limulus-System; höhere Dosen führen in der Regel zu einer zunehmenden Inaktivierung von LPS und/oder Lipid-A und/oder Glukanen im Plasma.

Des weiteren ist bei der Durchführung des erfindungsgemäßen Verfahrens zu beachten, daß nach der Durchführung der Oxidationsreaktion nicht zu große Mengen an Oxidans in der Probe zurückbleiben. So sind die Limulus-Faktoren C, B, A nur bis zu einem Zusatz von etwa 5 $\mu$mol Chloramin-T® pro ml Plasma-Probe resistent (im Gegensatz zu typischen Säugetier-Serinproteasen, wie beispielsweise Trypsin oder Plasmin, welche um mindestens das 10-fache resistenter sind, weswegen nach EP-A-297,597 kein Antioxidans zugesetzt wird). Daher sollte nach der Durchführung der Oxidation die verbliebene Menge des Oxidans gegebenenfalls inaktiviert werden, beispielsweise durch einen Quencher für Singulett-Sauerstoff. So wird beispielsweise bei Einsatz von 60 mM Chloramin-T® die nach der Oxidationszeit verbliebene Menge an Oxidans durch einen Singulett-Sauerstoff-Quencher, z.B. Ascorbat oder vorzugsweise Methionin, antagonisiert, bevor die Limulusfaktoren dem Reaktionsansatz zugesetzt werden oder Singulett-Sauerstoff-Quencher und Limulus-Faktoren werden gleichzeitig zugesetzt.

Bevorzugte Mengen an Oxidans, insbesondere Chloramin, nach Schritt a) in der Probe bewegen sich im Bereich von 20 bis 100 $\mu$mol pro ml Plasma-Probe.

Bei dem in Schritt b) eingesetzten Limulus Amöbocyten - Faktoren kann es sich um an sich bekannte Reagenzien handeln, die in der Regel gewonnen werden durch Lyse von Limulus-Amöbocyten (sogenanntes LAL), die allerdings auch gentechnisch hergestellt werden könnten. Vorzugsweise ist hier das Pyrochrome®-Reagenz einzusetzen, welches vergleichsweise zum Coamatic® sensitiver für LPS ist. Hinweise auf LAL innerhalb dieser Beschreibung sind so zu verstehen, dass auch anders hergestellte Limulus Amöbocyten - Faktoren eingesetzt werden können.

In Schritt b) können einzelne aber auch alle Limulus Amöbocyten - Faktoren eingesetzt werden. Bevorzugt setzt man eine Kombination der Faktoren A, B und C ein.

Die Bestimmung der Aktivierung mindestens eines der Limulus Amebozyten (LA)-Faktoren kann in an sich bekannter Weise erfolgen, beispielsweise durch die Ermittlung der Gelierungs-Zeit durch die LA-Faktoren, durch die Ermittlung der durch LA-Faktoren erzeugten Menge an Coagulin oder vorzugsweise durch die Ermittlung der Menge der durch LA-Faktoren freigesetzten Chromophors aus einer durch aktivierte LA- Faktoren spaltbaren chromogenen Verbindung.

Bei den erfindungsgemäß einzusetzenden Proben kann es sich um beliebige biologische Flüssigkeiten handeln, in denen aktive Lipopolysaccharide und/oder Lipid-A und/oder Glukane vorliegen können. Beispiele dafür sind Blut, Blutprodukte, Liquor, Urin, Saliva, Bronchoalveoläre Lavage, Aszites, Perikarderguß, Pleuraerguß, Milch, Gewebeflüssigkeiten, Lymphflüssigkeit und insbesondere Plasma. Weiterhin einzusetzende Proben sind Produkte, vorzugsweise industriell hergestellt, welche Anti-Limulus Faktoren enthalten, beispielsweise Plasmaprotein-Therapeutika wie Albumin oder Antithrombin III.

Ferner wurde gefunden, daß heparinhaltige biologische Flüssigkeiten trotz einer Oxidationsbehandlung die Meßergebnisse beeinflussen können. So ist beispielsweise selbst bei einer Oxidation mit 1 Teil 60 mmol/l Chloramin-T® pro 1 Teil Plasma die Matrix-Unabhängigkeit nur dann gewährleistet, wenn die Probe weniger als 1 IU/ml Heparin enthält.

Da Patientenplasma normalerweise selbst unter therapeutischer Heparinisierung weniger als 0,5 IU/ml Heparin enthält, können nahezu alle Patientenplasmen auf reaktives LPS und/oder Lipid-A und/oder Glukan untersucht werden.

Proben mit mehr als 1 IU/ml Heparin, wie sie beispielsweise bei Proben von Patienten unter Hämodialyse oder unter Herzlungenmaschinen-Operation mit bis zu etwa 10 IU/ml Heparin vorkommen, müssten mit hohen Dosen an Oxidationsmitteln, beispielsweise mit 100 $\mu$mol Chloramin-T® pro ml Plasma-Probe oxidiert werden, um vollständige Matrixunabhängigkeit zu gewährleisten oder das Heparin müsste durch Zusatz eines Heparininhibitors wie Polybren® neutralisiert werden.

Vorzugsweise arbeitet man mit vergleichsweise niedrigen Konzentrationen an Oxidationsmitteln, um die pathophysiologische Interaktion zwischen LPS bzw. Lipid-A bzw. Glukan und sensiblen menschlichen Zellen (= LPS-Reaktivität bzw. Lipid-A-Reaktivität bzw. Glukan-Reaktivität) besser zu imitieren. So ist eine Konzentration von etwa 60 mmol/l Chloramin-T® physiologischer als eine Konzentration von 120 mmol/l im Oxidansreagenz (Chloramin-Konzentrationen um die 10 mmol/l gibt es in der Mikroumgebung von aktivierten neutrophilen Granulozyten). Aus diesem Grunde werden vorzugsweise Konzentrationen von kleiner gleich 100 mmol/l, insbesondere kleiner gleich 60 mmol/l, Oxidationsmittel, vorzugsweise Chloramin-T®, im Oxidationsreagenz (bei 1:1 Verhältnis Plasmaprobe:Oxidans) verwendet, vorzugsweise bei Proben mit weniger als 1 IU/ml Heparin.

Gegebenenfalls kann der Probe ein Heparininhibitor, beispielsweise Polybrene® (Hexadimethrinebromide, Sigma, Deisenhofen, Deutschland) zugesetzt werden. Allerdings stimuliert Polybrene® das LPS/Limulus-System, was nicht im Sinne einer Imitation der LPS/Monozyten Reaktivität ist. Dennoch werden nach dem Stand der Technik vorzugsweise Heparinproben des Patienten (von typischerweise etwa 30-60 IU/ml Heparin im Plasma, beispielsweise Endo Tube ET, Chromogenix, Mölndal, Schweden) für den Limulus-Test eingesetzt.

Erfindungsgemäß bevorzugt eingesetzte Proben weisen bis zu 1 IU/ml Heparin auf, und enthalten einen Calcium-Komplexierer wie Citrat oder EDTA, wobei als Calcium komplexierende Verbindung Citrat bevorzugt ist. Gegebenenfalls kann ein Glukan-Inhibitor und/oder Glukan-Inaktivator (wie beispielsweise Zwittergent®, insbesondere Zwittergent® 3-14 (3-(N,N-Dimethylmyristylammonio)propansulfonat) und/oder beta-1,3-D-Glukanase (Helix pomatia oder Rhizoctonia solani) dem Test zugesetzt werden, um die Spezifität für LPS oder Lipid A zu erhöhen.

Die Auswertung des beanspruchten Nachweisverfahrens kann mit herkömmlichen Methoden, wie z.B. der Hyperbolic-Rate-Kinetik erfolgen. Ein weiterer Gegenstand der vorliegenden Erfindung ist allerdings ein Verfahren, bei dem die komplizierte Hyperbolic-Rate-Kinetik des Limulus-Tests in eine lineare Kinetik umgewandelt wird, was die Auswertung der Meßergebnisse extrem erleichtert.

Es wurde gefunden, daß durch Zusatz mindestens einer Guanidino-Verbindung, wie Arginin oder Guanidiniumkarbonat, und/oder eines Chaotropikums, beispielsweise eines Alkalimetallkations, wie K$^+$ , Na$^+$ oder Cs$^+$ oder eines Erdalkalimetallkations, wie Ca$^{++}$ oder Mg$^{++}$ vor oder während der Durchführung von Schritt c) oder zusammen mit den Limulus-Faktoren eine lineare Kinetik des Tests erreicht wird.

Insbesondere die Beseitigung der Matrixstörung aber auch die Linearisierung des Limulus-Testes und die Differenzierung zwischen Reaktivität von einerseits LPS bzw. Lipid-A und andererseits von Glukanen ermöglichen erstmals routinetaugliche Messungen von LPS und/oder Lipid-A und/oder Glukan, insbesondere von freiem LPS und/oder Lipid-A und/oder Glukanen, in Körperflüssigkeiten von Patienten, wie in deren Blut oder in Blutprodukten.

In einer weiteren Ausgestaltung des erfindungsgemäßen Verfahrens wird demzufolge eine Differenzierung zwischen Reaktivität von LPS bzw. Lipid-A und Glukanen und Reaktivität von Glukanen ermöglicht, so daß die Reaktivität beider Gruppen von Toxinen bestimmt werden kann. Dazu wird das oben beschriebene Verfahren in Anwesenheit und in Abwesenheit eines LPS-Inhibitors bzw. Lipid-A-Inhibitors durchgeführt. Die ermittelten Reaktivitäten werden voneinander subtrahiert und auf diese Weise die durch LPS und/oder Lipid-A spezifisch hervorgerufene Reaktivität ermittelt. Als LPS-Inhibitor bzw. Lipid-A-Inhibitor läßt sich beispielsweise Polymyxin-B (PMB, 1 mg = 7868 IU, Aerosporin, Euro OTC Pharma, Kamen, Deutschland; oder von Sigma-Fluka, Deisenhofen, Deutschland) einsetzen.

Zur Bestimmung der LPS- und/oder Lipid-A- und/oder Glukan-Reaktivität in der Probe wird diese zunächst durch ein Oxidans, wie ein Chloramin, voroxidiert, und die Reaktion wird vorzugsweise durch Zugabe eines Antioxidans beendet. Anschließend oder gleichzeitig zum Antioxidanszusatz wird der Limulus Test in an sich bekannter Weise durchgeführt und die Proben sowohl in Abwesenheit (Ansatz A) als auch in Anwesenheit eines Endotoxin-Inhibitors (Ansatz B) vermessen. Aus Ansatz A läßt sich die LPS+Lipid-A+Glukan Reaktivität der Probe ermitteln, während Ansatz B lediglich die Glukan-Reaktivität ergibt. Die Differenz der gemessenen Reaktivitäten, beispielsweise ermittelt als Differenz der Absorptionen A - B der freigesetzten Chromophore, ergibt den Gehalt an LPS- und Lipid-A-Reaktivität in Glukan-haltigen oder Glukan-freien Proben.

Die gemessenen und PMB-Eigenaktivität-korrigierten Absorptionen für Ansatz B (Bcorr) können in Reaktivitäten von Glukanen, beispielsweise in Zymosan A like Reactivity in $\mu$g/ml, umgerechnet werden.

Die Differenz der Absorptionen A - Bcorr können in Reaktivitäten von LPS und/oder Lipid-A, beispielsweise in LPS like Reactivity in ng/ml, umgerechnet werden.

Die Eichkurve des Tests ist bei Verwendung der oben beschriebenen Maßnahmen bis zu etwa 10000 ng/ml LPS in Normal-Plasma, insbesondere zwischen 200-300 ng/ml LPS, oder bis zu etwa 100 $\mu$g/ml Zymosan A in Normal-Plasma, insbesondere zwischen 2000-3000ng/ml = 2-3 $\mu$g/ml Zymosan A, linear. Diese Reaktivitäten beziehen sich auf gepooltes Normalplasma (NPG), welches mit diesen Mengen an LPS bzw. Lipid-A oder Glukan supplementiert wurde.

Bei Verwendung der Hyperbolic-Rate-Kinetic werden plasmatische LPS-Reaktivitäten

solche um die 1 ng/ml (d.h. 0.1 bis 5 ng/ml) nach ca. 60-120 min bei Raumtemperatur (RT, ca. 23˚C) nachweisbar:

solche um die 10 ng/ml (das heißt von 1 bis 50 ng/ml) nach ca. 30-60 min RT, solche um die 100 ng/ml (das heißt von 10 bis 500 ng/ml) nach ca. 15-30 min RT, solche um die 1000 ng/ml (das heißt von 100 bis 5000 ng/ml) nach ca. 8-15 min RT, solche um die 10000 ng/ml (das heißt von 1000 bis 50000 ng/ml) nach ca. 4-8 min RT, je nach Variabilität der produzierten LAL-Faktoren Charge Bezüglich der plasmatischen Glukan-Reaktivitäten ergeben sich vergleichbare Werte. Allerdings die ng/ml multipliziert mit dem Faktor 100.

Weist eine Probe höhere Werte an reaktivem LPS und/oder Lipid-A und/oder Glukan auf, so kann diese mit 3 - 10 %, bevorzugt 4-9 %, insbesondere 7% Albumin, vorzugsweise Humanalbumin, vorzugsweise in Phosphate Buffered Saline (PBS) verdünnt werden.

Die LPS- bzw. Lipid-A- oder Glukan-Reaktivität zeigt keinen "High-Dose-Inhibitory"-Effekt, d.h. extreme Konzentrationen an LPS bzw. Lipid-A oder Glukan, beispielsweise um die 10000 ng/ml bzw. 100 $\mu$g/ml Zymosan A, führen nicht zu einer Inhibition der Limulus-Reaktion.

Die Probe im hier beschriebenen Limulus-Test sollte vorzugsweise eine Proteinkonzentration haben, die in etwa der von menschlichem Plasma entspricht, d.h. 30 - 100 g/l, insbesondere 60 - 90 g/l.

Das erfindungsgemäße Verfahren, sowohl die Oxidationreaktion als auch die Limulusreaktion, werden bevorzugt bei Temperaturen zwischen 0˚C und 50 ˚C, insbesondere bevorzugt zwischen 15˚C und 45˚C, durchgeführt. Weiterhin wird das Verfahren bevorzugt unter physiologischen Bedingungen durchgeführt, insbesondere bei einem pH-Wert zwischen 6 und 10, bevorzugt zwischen 7,0 und 9,5, insbesondere zwischen 7,5 und 9,0. Gegebenenfalls wird der biologischen Flüssigkeit bzw. den verwendeten Reagenzien ein entsprechender Puffer zugesetzt.

Weiterhin wird auch ein geeignetes Testsystem zur Bestimmung der Lipopolysaccharid- und/oder Lipid-A- und/oder Glukan-Reaktivität beschrieben. Dieses umfaßt mindestens ein Oxidationsmittel und Limulus Amöbocyten-Faktoren zur Behandlung der Probe.

Besonders bevorzugt wird ein zur Bestimmung der Lipopolysaccharid- und/oder Lipid-A- und/oder Glukan-Reaktivität geeignetes Testsystem mit linearer Kinetik verwendet, das zusätzlich zu den oben genannten Reagenzien mindestens ein Linearisierungsreagenz enthält.

Weiterhin beschrieben wird ein Testsystem, das zusätzlich zu den oben genannten Reagenzien mindestens einen einen LPS- und/oder Lipid-A- Inhibitor enthält, um zwischen einerseits Lipopolysaccharid- und/oder Lipid-A- Reaktivität und andererseits Glukan-Reaktivität der Probe zu differenzieren.

Nachstehend wird eine typische Durchführung des erfindungsgemässen Tests zur Bestimmung der basalen LPS- und/oder Lipid-A- und/oder Glukan-Reaktivität beschrieben. Dieser wird vorzugsweise in zwei Ansätzen durchgeführt; beide Ansätze können jedoch auch getrennt voneinander durchgeführt werden.

Ansatz A (ergibt basale (LPS+Lipid-A+Glukane)-Reaktivität)

1 Teil Probe (z. B. 10 $\mu$l) wurde mit 1 Teil 15 - 200 mmol/l, bevorzugt 30 - 120 mmol/l, insbesondere 50-70 mmol/l Chloramin T (CT) in PBS oder durch Zusatz von 0,1-2 $\mu$molen, bevorzugt 0,3-1,2 $\mu$molen, insbesondere 0,5-0,7 $\mu$molen CT in 0,1 - 100 Teilen PBS für 10 min bei 37˚C (oder für 30 min bei Raumtemperatur) inkubiert. Dann wurden 1-5 Teile, bevorzugt 1 Teil, 100-230 mmol/l Methionin in $H_2O$ oder 1 - 11,5 $\mu$mole Methionin in $H_2O$ zugesetzt. Nach 0 - 5 min Raumtemperatur wurden 0,5 -5, vorzugsweise 2-3, Teile Limulus-Reagenz mit oder ohne chromogenes Limulus-Substrat zugesetzt (z. B. Pyrochrome®, welches zuvor in der vom Hersteller angegebenen Menge an Trispuffer rekonstituiert wurde, üblicherweise 3,2 ml 200 mmol/l Tris, pH 8, für den Limulus-Assay in Hyperbolic-Rate-Kinetik). Gegebenenfalls kann das Antioxidans und/oder der LPS-Inhibitor im Rekonstitutionsmaterial für die Limulusfaktoren oder bereits im Limulusfaktoren-Lyophilisat enthalten sein. Zur Erhöhung der Spezifität des chromogenen Substrates für Limulus-Faktoren kann gegebenenfalls das Antioxidans-Reagenz einen TrypsinInhibitor, insbesondere Aprotinin, vorzugsweise in einer Konzentration von 100-10000 KIU/ml, insbesondere 200-2000 KIU/ml enthalten und/oder Aprotinin-Dosen von 100-10000 KIU, insbesondere 200-2000 KIU pro ml Probe zu dem Reaktionsansatz zugesetzt werden.

Die Absorption wurde nach sofort bestimmt (BASE-value) und nach 1 - 10 min (bei Raumtemperatur). Dadurch wurde ein eventuell vorhandener linearer Extinktions-Kontroll-Anstieg (delta $A_c$) ermittelt. Falls die Probe Trypsin enthielt oder eine Trypsin-ähnliche Aktivität aufwies, wie bei einer Pankreatitis (ca. 10 - 100 ng Trypsin/ml) mußte die hier eventuell ermittelte lineare delta $A_c$ / min später von allen im Hyperbolic-Rate-Assay ermittelten Werten abgezogen werden.

Entweder wurde nun bei Raumtemperatur in 2-15 minütigem Abstand der Extinktions-Anstieg (delta A) bestimmt (Hyperbolic-Rate-Kinetik), oder nach 0,5 - 300 Minuten, bevorzugt 5 - 100 Minuten, insbesondere 10 - 50 Minuten, besonders bevorzugt 15 - 30 Minuten (RT) und/oder wenn beim 10 oder 100 ng/ml LPS-Reaktivitäts-Standard eine delta A von 3 - 30%, insbesondere ca. 15 % der maximal erreichbaren delta A auftrat, wurden 1-20 Teile, vorzugsweise 5-10 Teile, insbesondere 5 Teile Linearisierungsreagenz zugesetzt, bestehend aus einer Guanidino-Verbindung wie Arginin und/oder einem Chaotropikum wie Alkalikationen wie $K^+$, $Na^+$ oder $Cs^+$ oder Erdalkalikationen wie $Ca^{++}$ oder $Mg^{++}$ (finale Konzentration vorzugsweise 75-750 mmol/l; bevorzugte Konzentration im Zusatz = 100-1000 mmol/l, mit gegebenenfalls 0,1 - 2 mmol/l finaler Konzentration an chromogenem Substrat) und es wurde die delta A / t bestimmt. Die Zugabe von Substrat erfolgte vorzugsweise dann, wenn zuvor kein Substrat im Limulus-Reagenz enthalten war.

Der Zusatz von Linearisierungsreagenz transformierte die hyperbole in eine lineare Kinetik.

Gegebenenfalls wurden bis zu 50 mmol/l, insbesondere bis zu 30 mmol/l, Arginin (Spezifitätsreagenz) bereits in der ersten LAL-Inkubationsphase zugesetzt, da Arginin die Spezifität des Testes erhöhte, allerdings auch die Reaktionsgeschwindigkeit herabsetzte.

Statt Arginin kann erfindungsgemäß sowohl beim Linearisierungsreagenz als auch beim Spezifitätsreagenz eine andere guandinogruppentragende Substanz gewählt werden, wie z. B. Guanidiniumkarbonat, der pH Wert dieser Reagenzien sollte zwischen 7 und 10, insbesondere zwischen 7,5 und 9,5, liegen.

Ansatz B (ergibt basale Glukane-Reaktivität)

Es wurde wie bei Ansatz A vorgegangen mit der Änderung, daß zusätzlich Polymyxin B eingesetzt wurde und zwar 0,01 - 100 mg/ml Plasma-Probe, bevorzugt 0,1 - 10 mg/ml Plasma-Probe, insbesondere 0,2-5 mg/ml Plasma-Probe, besonders bevorzugt 1-3 mg/ml Polymyxin B (entsprechend 10-30 $\mu$g Polymyxin B pro 10 $\mu$l Plasma; 1 mg=7868 U).

15000 U/ml Polymyxin B neutralisierte bis zu 10000 ng/ml LPS-Reaktivität im gepoolten Normalplasma im hier beschriebenen Limulus-System.

Der für Normalpool (ohne Zymosan A) ermittelte delta A Wert wurde von allen im Ansatz B ermittelten delta A Werten abgezogen. Hierbei handelte es sich um eine durch den Polymyxin B - Zusatz erzeugte delta A, die von allen B-Werten abgezogen werden mußte.

Hatte der Patient nach dieser Korrektur der im Ansatz B ermittelten Werte positive delta A-Werte, so handelte es sich hierbei um die durch Glukane hervorgerufene Reaktivität.

Wies die Probe weniger als 50 ng/ml LPS Reaktivität auf, so konnte der Ansatz B auch mit 10-fach geringerem Zusatz an Polymyxin-B durchgeführt werden, d.h. besonders bevorzugt 0,1-0,3 mg/ml Plasma-Probe. Dies führte zu einer Verbesserung der Sensitivität des Testes.

Die im B-Ansatz ermittelten und korrigierten Werte (PMB-Base-Line = Aktivität in Normalplasma) sind von denen im A-Ansatz abzuziehen. Auf dieses Weise ermittelte man die LPS-Reaktivität im Untersuchungsmaterial

$$[LPS+Lipid\text{-}A+Glukane] - [Glukane] = [LPS+ Lipid\text{-}A].$$

Ansatz C (ergibt Trypsin-like-activity)
wie A, Linearisierungsreagenz wurde vor (oder gegebenenfalls bis zu 3 min nach) dem Limulus-Reagenz zugesetzt; die Inkubation nach Limulus-Reagenz-Zusatz entfiel.

Ansatz C ergibt die Trypsin-like-Activity (TLAC), im Normalfall nicht vorhanden; z.B. bei Pankreatitis, Intensivpatienten mit Gerinnungsaktivierung, gekühlten PlasmaProben oder bei nicht frischem Limulus-Reagenz positiv. Die TLAC ausgedrückt in mA/min muß von allen in A und B ermittelten Werten abgezogen werden.

Als Standards für das erfindungsgemäße Verfahren wurden beispielsweise gepooltes Normal-Plasma (oder 7% Humanalbumin) mit einer basalen LPS-Reaktivität (BL) von 0,2-2 ng/ml LPS, insbesondere von 0,4-1,2 ng/ml LPS, gemessen, das mit 0, 1, 10, 100, 1000, 10000 ng/ml LPS supplementiert wurde. Diese Eichstandards hatten definitionsgemäß LPS-Reaktivitäten wie 0+BL, 1 +BL, 10+BL, 100+BL, 1000+BL, 10000+BL ng/ml LPS in Normalplasma. Weitere Eichstandards hatten 0, 0.1, 1, 10, 100 µg/ml Zymosan A. Gegebenenfalls kann auch noch mit einer Trypsin-Kontrolle (beispielsweise 100 ng/ml Trypsin in 7 % Albumin-PBS) standardisiert werden, um den Gehalt an aktivem Trypsin in der Probe zu ermitteln.

Als Standards wurden Substrate gewählt, die eine Matrix - also eine Analyt-Umgebung - aufwiesen, die derjenigen Umgebung ähnelte, in welcher der Analyt eingebettet war. Geeignetes Probenmaterial sind insbesondere Citrat- oder EDTA-Plasmen. Bei der Untersuchung von EDTA-Proben sollten die Standards ebenfalls EDTA enthalten.

Soll eine Probe mit einer von Plasma verschiedenen Matrix untersucht werden, z.B. Liquor cerebrospinalis, Urin, bronchoalveoläre Lavage, Milch, ein Blutprodukt, ein kommerzielles Faktorenkonzentrat oder Saliva (Saliva enthält physiologischerweise 100-500 ng/ml LPS-Reaktivität, was ein Grund dafür ist, weswegen der Limulus-Test so störanfällig ist), so sollte die Probe mit Plasmapool oder gegebenenfalls mit 7 % Humanalbumin auf ungefähre Plasmamatrix durch eine Verdünnung von 1+2 bis 1+100, insbesondere von 1+4 bis 1 +9, gebracht werden, bevor sie im hier beschriebenen Test untersucht wird.

Alternativ kann eine Additionskalibration durchgeführt werden, d.h. Zusatz bekannter Mengen an LPS oder Lipid-A oder Glukan zu einer Probe, beispielsweise ein kommerzielles Antithrombin III Medikament, und Bestimmung der LPS- oder Lipid-A-oder Glukan-Reaktivität in der supplementierten und der nichtsupplementierten Probe. Die Absorptions-Differenz entspricht der zugesetzten LPS- oder Lipid-A- oder Glukan-Menge und ist ein Maß für die basale LPS-, Lipid-A- oder Glukan-Menge.

Normales Plasma hat ca. 0,8 ng/ml LPS-Reaktivität (Mittelwert=MV; d.h. es reagiert so, als hätte man einem Plasma ohne LPS-Reaktivität 0,8 ng/ml LPS hinzugesetzt). 1 Standardabweichung (SD) = 0,4 ng/ml LPS Reaktivität. Die intra-assay Variationskoeffizienten liegen unter 10 %.

Septische Patienten haben bis zu ca. 200 ng/ml, vereinzelt > 200 ng/ml LPS-Reaktivität und/oder bis zu 10 µg/ml, vereinzelt > 10 µg/ml Glukan-Reaktivität im Plasma. Die untere Nachweisgrenze des erfindungsgemäßen Testes beträgt ca. 0,1 ng/ml LPS-Reaktivität und/oder ca. 0.1 µg/ml Glukan-Reaktivität im Plasma.

Nur etwa 1 % des zu Normalpool zugesetzten LPS reagiert im erfindungsgemäßen Verfahren so wie LPS bei Zusatz zu 7 % Humanalbumin reagiert, d.h. das Plasma enthält LPS-Inhibitoren, inhibiertes LPS wird im vorliegenden Verfahren nicht miterfasst. Nur etwa 10 % des zu Normalpool zugesetztem Glukan reagierte im erfindungsgemäßen Verfahren so wie Glukan bei Zusatz zu 7 % Humanalbumin reagiert, d.h. das vorliegende Verfahren erfasst nicht neutralisierte Glukane.

Plasmaproben sollten möglichst unverdünnt eingesetzt werden, da so die pathophysiologische LPS- und/oder Lipid-A- und/oder Glukan-Reaktivität am besten imitiert wird.

Die Inkubationszeiten bei Raumtemperatur (RT, ca. 23°C) werden 3-fach verkürzt, wenn bei 37°C inkubiert wird. Da das Limulus-System extrem hitzesensibel ist, sollte die Inkubationstemperatur 37°C nach Möglichkeit nicht überschreiten.

Ausgewertet werden nur delta A Werte unter 45% der maximal erreichbaren delta A.

In der nachstehenden Auflistung wird die beste Ausführungsform für Ansatz A dargestellt. Danach werden die besten Ausführungsformen für Ansätze B und C beschrieben.

Bestimmung der LPS und/oder Lipid-A und/oder Glukan-Reaktivität (Ansatz A)

10 µl Probe
10 µl Oxidans-Reagenz (60 mM CT)
10 min 37°C (oder 30 min RT)
10 µl Antioxidans-Reagenz (230 mM Methionin)
3 min RT
30 µl Limulus-Reagenz (z. B. Pyrochrome®, rekonst. in 3,2 ml Trispuffer)
20 min RT
50 µl Linearisierungs-Reagenz (750 mM Arginin, pH 8,7)
Test mit Linearisierung der Kinetik:

delta A / t

Gegebenenfalls Test mit Linearisierung der Kinetik:

20 min RT oder 40 min RT

50 µl Linearisierungs-Reagenz (750 mM Arginin, pH 8,7)

delta A/t

Bestimmung der basalen Glukan-Reaktivität (Ansatz B)

Wie Ansatz A, Antioxidans-Reagenz enthält zusätzlich 15000 U/ml Polymyxin B

Bestimmung der Trypsin-like-activity (Ansatz C)

Test ohne Linearisierung: Wie Ansatz A, Bestimmung eines linearen delta A/t Anstiegs innerhalb der ersten 1-10 min RT in Ansatz A und B.

Test mit Linearisierung: Wie in Ansatz A, Linearisierungsreagenz wird vor (oder gegebenenfalls bis zu 3 min nach) dem Limulus-Reagenz zugesetzt; die 20 min oder 40 min RT -Inkubation vor der Zugabe des Linearisierungs-Reagenzes entfällt.

Wird bei 37˚ statt bei RT inkubiert, so verkürzen sich die Zeiten dreifach.

Zusätzlich zur basalen LPS- und/oder Lipid-A- und/oder Glukane-Reaktivität (Ansätze A, B) konnte die LPS- und/oder Lipid-A- und/oder Glukane-Reaktivität getestet werden, nachdem zusätzlich LPS oder Lipid-A oder Glukane zur Probe zugesetzt wurden. Damit wurde die individuelle Inhibitionskapazität gegenüber LPS und/oder Lipid-A und/oder Glukanen getestet.

Patienten mit geringer LPS/Lipid-A/Glukane-Inhibitionskapazität (z. B. wenig diesbezügliche Antikörper oder LBP im Plasma) sind empfindlicher für pathologische DIC. Dies wurde in den nachstehend beschriebenen Ansätzen D oder E getestet.

Ansatz D (ergibt addierte LPS-und/oder Lipid-A-Reaktivität=LPS-und/oder Lipid-A-Inhibitionskapazität)

Es wurde wie bei Ansatz A vorgegangen mit der Änderung, daß der Probe 0,5 Teile 1 - 1000, bevorzugt 2 - 50, insbesondere 10 ng/ml LPS zugesetzt wurden und gegebenenfalls vor Oxidation 1-60 min RT inkubiert wurde:

100 % der Norm = Anstieg um 5 ng/ml LPS-Reaktivität; SD = 50 %, Patienten haben bis zu etwa 1000 - 10000 % der Norm.

Ansatz E (ergibt addierte Glukan-Reaktivität=Glukan-Inhibitionskapazität)

Es wurde wie bei Ansatz A vorgegangen mit der Änderung, daß, der Probe 0,5 Teile 0,1 - 100, bevorzugt 0,4 - 4, besonders bevorzugt 1 $\mu$g/ml Zymosan A zugesetzt wurde:

100 % der Norm = Anstieg um 0,5 $\mu$g/ml Glukan-Reaktivität; SD = 20 %, Patienten haben bis zu ca. 100 - 1000 % der Norm.

Die nachstehenden Beispiele erläutern das erfindungsgemäße Verfahren, ohne dieses zu begrenzen.

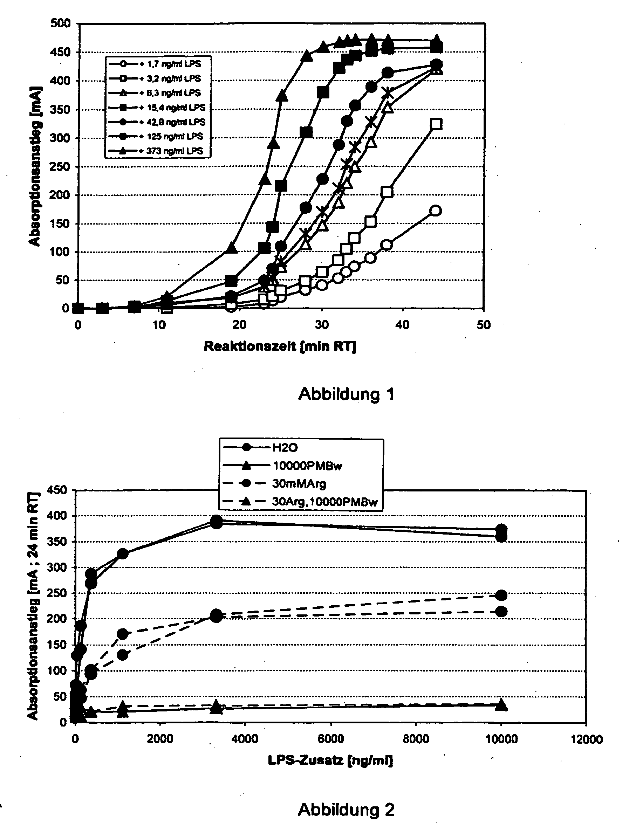

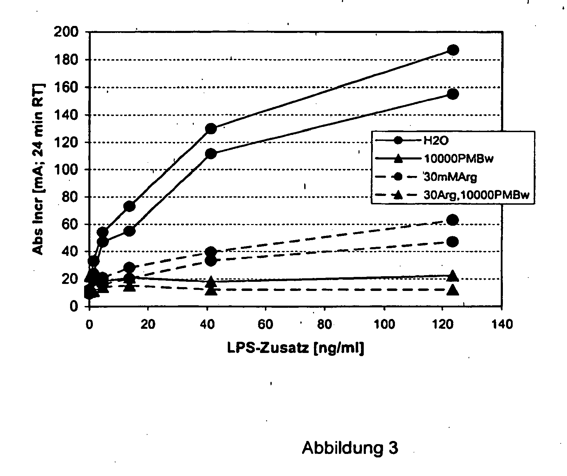

In den Abbildungen 1 bis 24 sind unter Einsatz des erfindungsgemäßen Verfahrens erhaltenen Meßergebnisse dargestellt.

Beispiel 1 (Ansatz A)

10 $\mu$l Plasma des Patienten und Plasma-Standards mit 0, 1, 10, 100, 1000, 10000 ng/ml LPS-Reaktivität wurden in Doppelbestimmung mit 10 $\mu$l 60 mM Chloramin-T® in PBS für 10 min bei 37˚C (oder 30 min bei Raumtemperatur=RT) inkubiert. Dann wurden 10 $\mu$l 230 mM Methionin in $H_2O$ zugesetzt. Nach 3 min RT wurden 30 $\mu$l Limulus-Reagenz mit chromogenem Limulus-Substrat S-2834® zugesetzt (Pyrochrome®). Die Absorption wurde nach 2 min (RT; BASE-value) und nach 4 min (RT; Trypsin-Kontrolle für Hyperbolic-Rate-Kinetik) bestimmt. Nach 20 min (RT) wurde das Linearisierungreagenz zugesetzt, hier 50 $\mu$l 750 mM Arginin, pH 8,7 (finale Konzentration 341 mM) und die delta A / t bestimmt.

Dieser Wert entsprach der (LPS+Lipid-A+Glukan)-Reaktivität der Probe.

Beispiel 2 (Ansatz B)

Es wurde wie bei Ansatz A vorgegangen mit der Änderung, daß mitgemessene Standards 0, 1, 2, 4, 8, 16, 32 $\mu$g/ml Zymosan A in gepooltem Glukan-freiem Normalplasma waren. Das Methionin-Reagenz enthielt zusätzlich 15000 U/ml Polymyxin B (PMB). Der für Normalpool (ohne Zymosan A) ermittelte delta A Wert wurde von allen im Ansatz B ermittelten delta A Werten abgezogen (PMB-Korrektur). Der PMB-korrigierte B-Wert entsprach der Glukan-Reaktivität der betreffenden Probe. Die im B-Ansatz ermittelten und PMB-korrigierten Werte waren von denen im A-Ansatz abzuziehen: so erhielt man die LPS-+Lipid-A-Reaktivität der Probe [LPS+Lipid-A+Glukane] - [Glukane] = LPS+Lipid-A.

Beispiel 3 (Ansatz C)

Es wurde wie bei Ansatz A vorgegangen mit der Änderung, dass das Linearisierungsreagenz vor dem Limulus-Reagenz zugesetzt wurde und der Absorptionsanstieg (delta A) direkt bestimmt wurde.

Beispiel 4 (Ansatz D)

Es wurde wie bei Ansatz A vorgegangen mit der Änderung, daß der Probe 5 $\mu$l 10 ng/ml LPS zugesetzt wurden und für 10 min RT vor der Oxidation inkubiert wurde.

Beispiel 5 (Ansatz E)

Es wurde wie bei Ansatz A vorgegangen mit der Änderung, daß der Probe 5 $\mu$l 1 $\mu$g/ml Zymosan A zugesetzt wurden und für 10 min RT vor der Oxidation inkubiert wurde.

Beispiel 6: Hyperbolic Rate-Kinetik mit Pyrochrome®-Reagenz

1 ml gepooltes Patientenplasma (Citrat; enthaltend 1,8 ng/ml LPS Reaktivität) mit normaler plasmatischer Gerinnung wurde mit 0 - 10000 ng/ml LPS (Sigma, *E. coli B5*, gelfiltriert) oder mit 1 $\mu$g/ml Zymosan A (Sigma) supplementiert und mit 1 ml 60 mM Chloramin-T® (=CT) in PBS 10 min (37˚C) in pyrogenfreien U-Mikrotiterplatten inkubiert. 1 ml 230 mmol/l Methionin in $H_2O$ wurde zugesetzt (vergleichbar 3,33 $\mu$l Probe + 3,33 $\mu$l 60 mM CT + 3,33 $\mu$l 230 mM Methionin) und nach 3 min (Raumtemperatur) wurden je 10 $\mu$l dieser Mischung mit 5 $\mu$l $H_2O$ oder 10000 U/ml Polymyxin B (Euro OTC Pharma, Kamen, Deutschland) in $H_2O$ und 5 $\mu$l $H_2O$ (Ansatz 1 a) oder 240 mM Arginin (Ansatz 1 b), pH 8,7 (30 mmol/l final) und 20 $\mu$l Pyrochrome®-Reagenz bei Raumtemperatur inkubiert.

Die Absorptions-Werte des gemäß Beispiel 6 durchgeführten Versuchs wurden nach 0 - 44 min (RT) bei 405 nm bestimmt. Es wurden die in Abbildungen 1-3 dargestellten Werte ermittelt.

Aus Abbildung 1 ist zu erkennen, dass bei Einsatz von 20 $\mu$l Limulus-Reagenz (mit chromogenem Substrat-Kolyophilisat; Probenmenge = 3,3 $\mu$l) sich eine maximale delta A von 470 mA ergibt. Eine delta A von 100 mA (entsprechend ca. 20 % der delta A max.) resultiert bei 1,7 ng/ml LPS-Reaktivität im Plasma nach 37 min RT, bei 3,2 ng/ml LPS-Reaktivität nach 32 min RT, bei 6,3 ng/ml LPS-Reaktivität nach 26 min RT, bei 15,4 ng/ml LPS-Reaktivität nach 24 min RT, bei 42,9 ng/ml LPS-Reaktivität nach 23 min RT, bei 125 ng/ml LPS-Reaktivität nach 22 min RT, bei 373 ng/ml LPS-Reaktivität nach 18 min RT.

Aus Abbildung 2 ist zu erkennen, dass bei Verwendung von 3,3 $\mu$l Probe, 3,3 $\mu$l Oxidans, 3,3 $\mu$l Methionin, 10 $\mu$l $H_2O$ oder Arginin und 20 $\mu$l Pyrochrome®-Reagenz und 24 min RT -Inkubation sich eine Linearität des Limulus-Assays bis zu ca. 100-300 ng/ml LPS-Zusatz zu Normalplasma zeigt, entsprechend 300 ng/ml LPS-Reaktivität. Ohne Einsatz von 30 mM finalem Arginin als Spezifitätsreagenz ergibt sich lediglich eine ca. 5 % Verminderung der Extinktion bei 10000 ng/ml LPS-Reaktivität gegenüber 3333 ng/ml LPS-Reaktivität (•-• in Doppelbestimmung), d.h. der Test hat keinen high-dose-inhibitory-effect.

Wird 30 mM Arginin als Spezifitätsreagenz verwendet (•- -• in Doppelbestimmung), so verlangsamt sich die Limulus-Reaktion, eine delta A von 200 mA nach 24 min RT wird ohne 30 mM Arginin durch Zusatz zu Plasma von ca. 100 ng/ml LPS, mit 30 mM Arginin durch Zusatz zu Plasma von ca. 3000 ng/ml LPS erreicht. Zusatz von 50 $\mu$l 10000 U/ml PMB inhibiert jeglichen LPS-Zusatz bis zu 10000 ng/ml (▲).

Abbildung 3 zeigt den Hyperbolic-Rate-Assay in diesem Experiment im niedrigen Bereich des LPS-Zusatz zu Plasma.

In Abbildung 4 wird die Inhibition von LPS durch Polymyxin B und die basale Aktivität von Polymyxin B selbst auf das Limulus-System dargestellt. Alle mit Polymyxin B erhaltenen Werte delta A-Werten mussten folglich korrigiert werden, indem der basale delta A- Wert für Glukan-freies Plasma abgezogen wurde. Wird der so korrigierte Polymyxin-B Wert vom Wert im Ansatz A (vergleiche Abbildung 1) abgezogen, so erhielt man die LPS- und/oder Lipid-A- Reaktivität des Patienten.

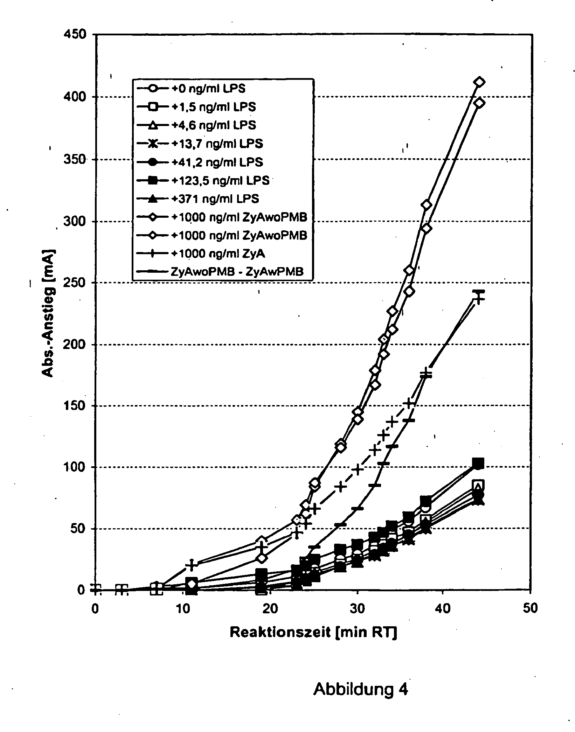

Aus Abbildung 4 ist zu erkennen, dass 5 $\mu$l 10000 U/ml PMB Zusatz zu einem delta A von ca. 70 mA nach 44 min RT selbst in Glukane-freiem Plasmapool (O) führt. Die Abbildung 4 zeigt, dass LPS-Zusatz bis zu 371 ng/ml (▲, ■, •, ×, △, □) zu

Normalplasma vollständig neutralisiert wird durch PMB in den ersten 44 min RT Limulus-Reaktions-Zeit. 1000 ng/ml Zymosan A mit PMB (+) oder ohne PMB (◊) kann störungsfrei nachgewiesen werden; (-) zeigt die Differenz von 1000 ng/ml Zymosan A (ZyA) mit PMB (+) minus die PMB-Eigenkontrolle (○).

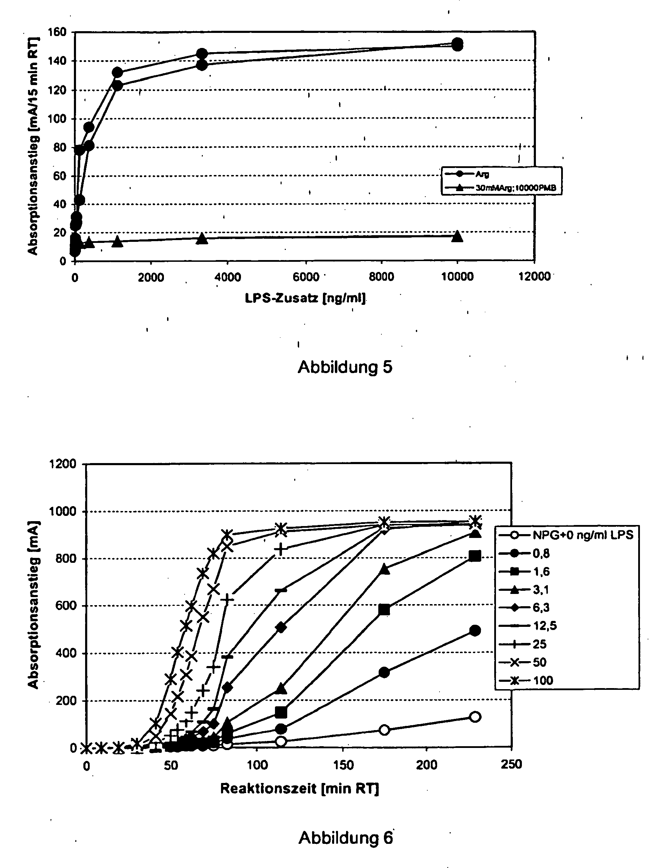

In Abbildung 5 wird gezeigt, wie bei der Durchführung des erfindungsgemäßen Verfahrens eine linear Rate-Kinetik erzeugt werden kann. So wurde beispielsweise die Kinetik von Ansatz 1 b durch Zusatz von 50 $\mu$l 750 mM Arginin, pH 8,7 (finale Arginin-Konzentration 30+417=447 mmol/l) 23 min bei RT nach Zusatz des LAL-Reagenzes linearisiert.

Der in Abbildung 5 dargestellte 30 mM final Arginin-Ansatz wurde mit 50 $\mu$l 750 mM Arginin linearisiert (•). Es ergeben sich lineare Extinktionsanstiege, hier als Anstieg /15 min RT dargestellt. Es ergibt sich eine Test-Linearität bis zu ca. 300 ng/ml LPS-Reaktivität. Selbst 10000 ng/ml LPS-Reaktivität führt nicht zu einem High-Dose-Inhibitory-Effect. 5 $\mu$l 10000 U/ml PMB-Zusatz neutralisiert die LPS-Reaktivität bis zu 10000 ng/ml (▲), wenn der Linearisierungszeitpunkt 23 min RT ist.

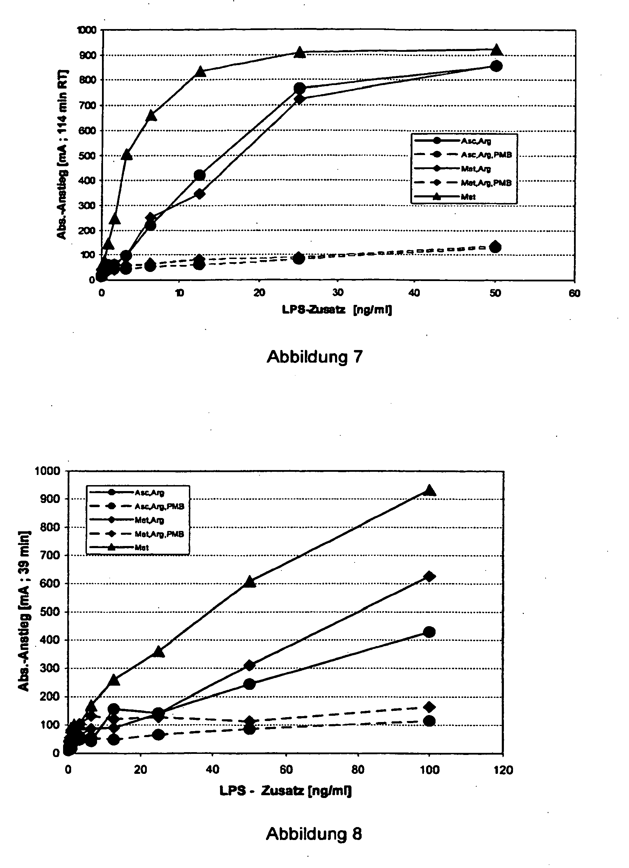

In den Abbildungen 6 und 7 wird die Durchführung des erfindungsgemäßen Verfahrens unter Einsatz eines Hyperbolic-Rate Assay mit Coamatic®-Reagenz im Vergleich zu einem Hyperbolic-Rate Assay mit Pyrochrome®-Reagenz dargestellt.

Dazu wurden 10 $\mu$l Normalplasmapool, supplementiert mit 0 -100 ng/ml LPS mit 10 $\mu$l 60 mmol/l CT 10 min lang bei 37˚C inkubiert. 10 $\mu$l 200 mmol/l Methionin mit 2000 U/ml Polymyxin B (Sigma) wurden zugesetzt und nach 3 min RT wurden 30 $\mu$l LAL-Reagenz Coamatic® (Chromogenix, Mölndal, Schweden) zugesetzt und bei 23˚C oder 37˚C inkubiert.

Abbildung 6 zeigt, dass 20 % der delta A max. ca. 200 mA sind; diese delta A wird erreicht nach 40 min RT bei + 100 ng/ml LPS, nach 50 min RT bei + 50 ng/ml LPS, nach 60 min RT bei + 25 ng/ml LPS, nach 70 min RT bei 12,5 ng/ml LPS, nach 75 min RT bei + 6,3 ng/ml LPS, nach 100 min RT bei + 3,1 ng/ml LPS, nach 120 min RT bei + 1,6 ng/ml LPS, nach 140 min RT bei + 0,8 ng/ml LPS, nach ca. 270 min RT bei + 0 ng/ml LPS. Das Coamatic®-Reagenz ist wesentlich insensitiver für LPS als das Pyrochrome®-Reagenz.

In Abbildung 7 wird der Einfluß der Zugabe von Ascorbat (Asc) und Methionin (Met) im erfindungsgemäßen Verfahren dargestellt. Dazu wurde Normalpool mit 0-100 ng/ml LPS supplementiert. 10 $\mu$l davon wurden mit 10 $\mu$l 60 mmol/l CT 10 min bei 37˚C inkubiert. 10 $\mu$l 210 mmol/l Ascorbat oder 210 mmol/l Methionin mit oder ohne 210 mmol/l Arginin jeweils mit oder ohne 2000 U/ml Polymyxin B wurden zugesetzt. Nach 3 min bei RT wurden 30 $\mu$l LAL-Reagenz Coamatic® zugesetzt und bei 37˚C inkubiert. Der Anstieg der Absorption wurde gemessen. Man erkennt, daß sich sowohl Ascorbat als auch Methionin eignen, um die Oxidation zu neutralisieren.

Aus Abbildung 7 ist zu erkennen, dass der Wert für + 0 ng/ml LPS erst richtig beurteilt werden kann bei Verlängerung der Inkubationszeit auf über 100 min RT. Die verlängerte Inkubationszeit erlaubt eine bessere Messung der LPS-Reaktivitäten unter 5 ng/ml LPS.

Abbildung 8 zeigt, dass 2000 U/ml PMB nur bis zu ca. 20 ng/ml LPS-Reaktivität vollständig inhibieren, dass 50 ng/ml LPS trotz 2000 U/ml PMB zu einem delta A von zusätzlich 100 mA führen, der einer LPS-Reaktivität von + 1 ng/ml entspricht. 30 mM Arginin inhibiert das LAL-System bei Coamatic wesentlich weniger als bei Pyrochrome.

Aus den Abbildungen 7 und 8 erkennt man, daß das Coamatic®-Reagenz eine wesentliche langsamere Kinetik aufweist als das Pyrochrome®-Reagenz, d.h.

Coamatic-Reagenz enthält weniger Faktor C aber mehr chromogenes Substrat als Pyrochrome.

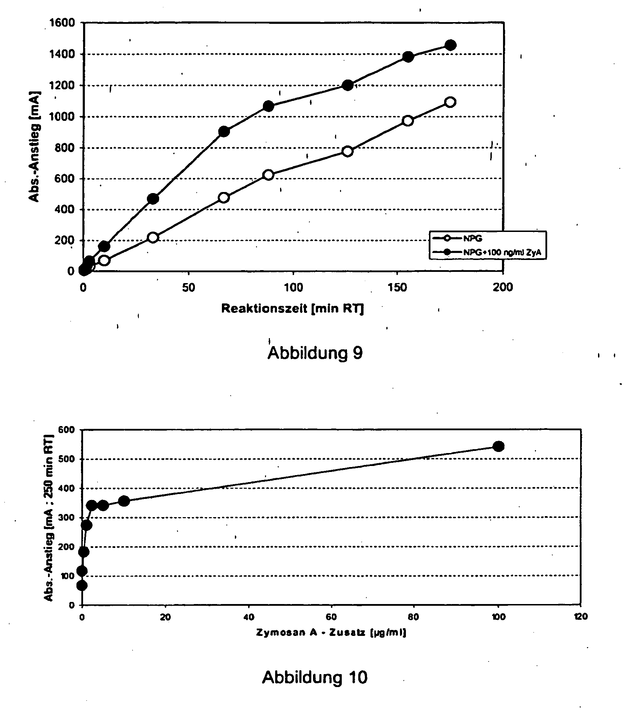

In Abbildung 9 wird ein erfindungsgemäßes Verfahren dargestellt, bei dem die Reaktivität von 100 ng/ml Zymosan A in Normalplasma ermittelt wurde. Dazu wurden 10 $\mu$l Normalpoolplasma supplementiert und mit 0 oder 100 ng/ml Zymosan A mit 10 $\mu$l 60 mmol/l CT für 10 min bei 37˚C inkubiert. Dann wurden 10 $\mu$l 200 mmol/l Methionin mit 2000 U/ml Polymyxin B zugesetzt. Nach 3 min (RT) wurden 30 $\mu$l LAL-Reagenz Coatest® (Limulus-Faktoren nicht als Kolyophilisat mit einem chromogenen Substrat) zugesetzt. Nach 40 min bei 37˚C wurden 75 $\mu$l 750 mM Arginin, pH 8,7 und 25 $\mu$l 2 mmol/l CS-2834® zugesetzt und bei RT der Absorptionsanstieg gemessen. Aus Abbildung 9 ist zu erkennen, daß 100 ng/ml Zymosan deutlich gemessen werden konnten.

Abbildung 9 zeigt, dass der Maximalausschlag ca. 1800 mA beträgt, erreicht durch 25 $\mu$l 2 mM S-2834®, entsprechend 30 $\mu$l 1,7 mM chromogenem Substrat (CS), d.h. dass im Coamatic-Reagenz ca. 0,55 mM CS und im Pyrochrome-Reagenz ca. 0,33 mM CS als Kolyophilisat vorliegen.

Abbildung 10 zeigt die Ergebnisse eines erfindungsgemäßen Verfahrens, bei dem 10 $\mu$l Normalplasmapool supplementiert mit 0 - 100 $\mu$g/ml Zymosan A mit 10 $\mu$l 60 mmol/l CT 30 mmol/l NaHCO$_3$ für 30 min bei RT inkubiert wurden. Dann wurden 10 $\mu$l 215 mmol/l Methionin, 150 mmol/l Arginin, pH 8,7, und 1000 U/ml Polymyxin B zugesetzt und nach 3 min (RT) wurden 15 $\mu$l LAL-Reagenz Coamatic® zugesetzt und delta A gemessen. Der Test hier ist linear bis zu 2 $\mu$g/ml Zymosan A.

Abbildung 11 zeigt die Ergebnisse eines erfindungsgemäßen Verfahrens, bei dem 10 $\mu$l Normal-Plasma (Citrol; DadeBehring, Marburg, Deutschland) supplementiert mit 0 - 1000 ng/ml LPS mit 10 $\mu$l 60 mmol/l CT inkubiert wurden. Nach 30 min bei RT wurden 10 $\mu$l 210 mmol/l Arginin, 210 mmol/l Natrium-Ascorbat und 30 $\mu$l LAL-Reagenz Coamatic® zugesetzt und delta A bei 405 nm gemessen. Abbildung 11 zeigt die Kinetik bei Raumtemperatur.

Aus Abbildung 11 ist zu entnehmen, dass eine delta A von 200 mA, d.h. ca. 20 % der max. delta A, erreicht wird bei + 1000 ng/ml LPS nach 20 min RT, bei + 500 ng/ml LPS nach 25 min RT, bei + 250 ng/ml LPS nach + 30 min RT, bei + 125 ng/ml LPS nach 32 min RT, bei +63 ng/ml LPS nach 37 min RT, bei + 31 ng/ml LPS nach 45 min RT, bei + 8 ng/ml LPS nach 57 min RT, bei + 4 ng/ml LPS nach 72 min RT, bei + 1 ng/ml LPS nach 80 min RT, bei + 0 ng/ml LPS nach 108 min RT. Die LPS-Kinetik bei Coamatic ist folglich wesentlich langsamer als die bei Pyrochrome (Abb.1), insbesondere bei niedrig LPS-supplementierten Proben (hier um den Faktor 2-3). Abbildung 12 zeigt die Ergebnisse eines erfindungsgemäßen Verfahrens, bei dem 10 µl 0-20 % Humanalbumin supplementiert mit 5 ng/ml LPS mit 10 µl 60 mmol/l CT für 10 min bei 37°C inkubiert wurden. Sodann wurden 10 µl 200 mmol/l Methionin und 30 µl LAL-Reagenz Coamatic® zugesetzt und delta A bei RT gemessen. Man erkennt die starke Abhängigkeit des Limulus-Testes vom Proteingehalt der Probe.

Aus Abbildung 12 ist,zu erkennen, dass bei Ansteigen der Albumin-Konzentration der Probe von 0 % auf 1 %, die LPS-Reaktivität auf ca. 1/5 des Ausgangswertes abfällt. Wird die Albumin-Konzentration auf ca. 2 % erhöht, so ergibt sich wieder der Ausgangswert. Bei weiter steigender Albuminkonzentration von 2 % auf 10 % der Probe fällt die LPS-Reaktivität auf 1/3 des Ausgangswertes; eine weitere Steigerung der Albuminkonzentration der Probe von 10 % auf 20 % führt nicht zu einer Veränderung der LPS-Reaktivität der Probe. Albumin stabilisiert folglich LPS (z.B. gegenüber Bindung an die Plastikwand der Platte), inhibiert aber zugleich die LPS-Reaktivität mit Limulus-Faktoren.

Abbildung 13 zeigt die Ergebnisse eines erfindungsgemäßen Verfahrens, bei dem 10 µl Normalplasmapool (NPG) supplementiert mit 0 - 6667 ng/ml Zymosan A mit 10 µl 60 mmol/l CT für 10 min bei 37°C inkubiert wurden. Sodann wurden 10 µl 200 mmol/l Methionin und 30 µl LAL-Reagenz Coamatic® zugesetzt und delta A bei RT gemessen. Man erkennt die Linearität (Verhältnis von Glukane-Konzentration zu resultierender delta A) des Limulus-Testes für Glukane hier bis zu einer PlasmaKonzentration von etwa 2 µg/ml Glukane.

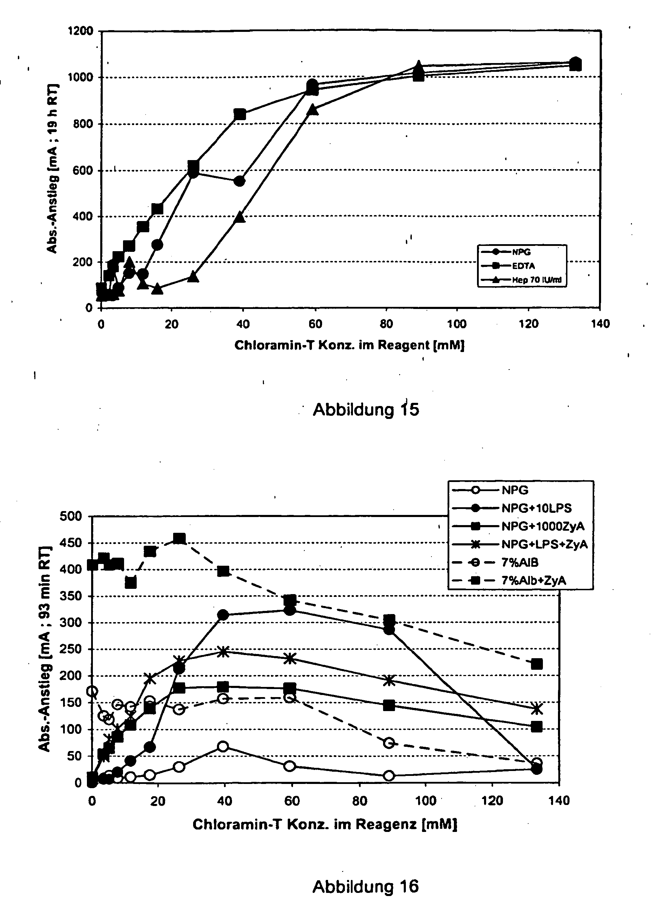

Abbildungen 14 und 15 zeigen die Ergebnisse eines erfindungsgemäßen Verfahrens, bei dem 10 µl Probe (NPG, NPG plus 3,2 mg/ml EDTA, NPG plus 70 IU/ml Heparin) supplementiert mit 0 oder 10 ng/ml LPS mit 10 µl 0-133 mmol/l CT in $H_2O$ für 10 min bei 37°C inkubiert wurden. Sodann wurden 10 µl 200 mmol/l Ascorbat und 30 µl LAL-Reagenz Coamatic® zugesetzt. Abbildung 14 (für 10 ng/ml supplementierte Plasmen) und Abbildung 15 (für unsupplementierte Plasmen) zeigen, daß CT - Konzentrationen von 40-80 mmol/l optimal sind, um den Heparineffekt auszuschalten.

Abbildung 14 zeigt, dass sich ohne Oxidation keine messbare LPS-Reaktivität in 50 min RT ergibt. 70 IU/ml Heparin in der Probe stimulieren die LPS-Reaktivität ca. 5-fach bei Verwendung eines Oxidans-Reagenzes mit ≥60 mM CT. Da das erfindungsgemäße Verfahren die pathophysiologische Reaktivität von LPS und/oder Lipid-A und/oder Glukanen mit humanen Rezeptoren für LPS und/oder Lipid-A und/oder Glukanen (z.B. CD 14 oder Phospholipasen von Monozyten) imitieren soll, werden erfindungsgemäß statt Heparin-Probenröhrchen - wie nach dem Stand der Technik vorzugsweise Citrat- oder EDTA- Monovetten für die Probennahme empfohlen.

Abbildung 15 zeigt, dass sich ohne Oxidation nur eine Limulus-Reaktivität von < 100 mA/19 h RT für Normalplasma ergibt. Zusatz von 40-80 mM CT erhöht diese mehr als 10fach. Mindestens 60 mM CT im Oxidationsreagenz sind notwendig, damit die LPS-Reaktivität von Heparinplasmen (70 IU/ml in Probe) der LPS-Reaktivität von normalem Citrat- oder EDTA-Pool gleichkommt.

Abbildung 16 zeigt die Ergebnisse eines weiteren erfindungsgemäßen Verfahrens, bei dem 10 µl 0-133 mmol/l CT in 100 mmol/l $NaHCO_3$, pH 8,5, für 10 min (37°C) inkubiert wurden mit 10 µl Poolplasma normaler plasmatischer Gerinnung (NPG) oder 7% Humanalbumin (Kabi, Stockholm, Schweden), jeweils supplementiert mit 0 oder 10 ng/ml LPS und/oder 1000 ng/ml Zymosan A (ZyA). Sodann wurden 50 µl 200 mmol/l Methionin und nach 3 min bei RT 30 µl LAL-Reagenz Coamatic® zugesetzt und delta A wurde nach 93 min bei RT bestimmt. Die optimale CT Konzentration in diesem Versuch liegt bei etwa 60 mmol/l.

Aus Abbildung 16 ist zu erkennen, dass für Normalplasma, unsupplementiert und LPS- oder ZyA- supplementiert, sich maximale LPS- oder Glukane-Reaktivitäten ergeben, wenn das Oxidans-Reagenz 40-80 mM CT enthält. Im Oxidans-Maximum ergibt Plasmapool (NPG) + LPS + ZyA eine ca. 30 % niedrigere Endotoxin-Reaktivität als NPG + LPS. Oxidation mit > 60 mM CT führen zu einem Abfall der Limulus-Aktivität der 7 % Humanalbumin-Probe, bei > 60 mM CT scheint ZyA zunehmend zerstört zu werden. LPS in Plasma wird durch Oxidation mit > 90 mM CT zunehmend zerstört.

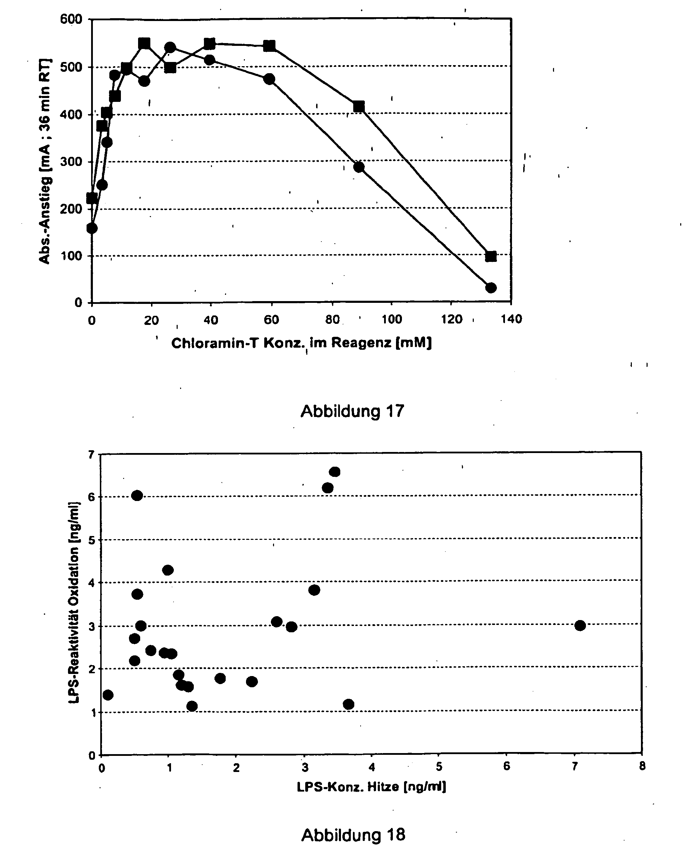

Abbildung 17 zeigt die Ergebnisse eines weiteren erfindungsgemäßen Verfahrens, bei dem 10 µl 0-133 mmol/l CT in 100 mmol/l $NaHCO_3$, pH 8,5 mit 10 µl 7% Humanalbumin supplementiert mit 10 ng/ml LPS (•) oder mit 10 ng/ml LPS und 1000 ng/ml Zymosan A (■) 10 min bei 37°C inkubiert wurden. Es wurden 50 µl 200 mmol/l Methionin zugesetzt und nach 3 min RT wurden 30 µl LAL-Reagenz Coamatic® zugefügt und delta A bei 405 nm gemessen. Abbildung 17 zeigt die Ergebnisse mit unterschiedlichen CT Konzentrationen, über 80 mmol/l CT inaktivierten Endotoxin.

Aus Abbildung 17 ist zu erkennen, dass sich maximale LPS-Reaktivität durch Oxidation mit 20-60 mM CT ergibt, dass höhere Oxidans-Konzentrationen zu einer verringerten LPS-Reaktivität führen und dass LPS in Albuminprobe durch Oxidation mit > 80 mM CT zunehmend zerstört zu werden scheint.

Abbildung 18 zeigt die Ergebnisse weiterer Verfahren, bei denen n=24 willkürliche EDTA-Plasmen von Patienten mittels Coamatic®-LAL Test analysiert wurden und mit Hitze (10-fache Verdünnung mit $H_2O$, 15 min bei 75 °C) und mit 60 mmol/l CT behandelt wurden. Aus Abbildung 18 ist zu erkennen, daß die Verfahren unterschiedliche Reaktivitäts-Ergebnisse liefern (Erhitzen: 1,8 ng/ml LPS +- 1,6 ng/ml; CT-Behandlung: 2,8 ng/ml +- 1,6 ng/ml; der Korrelationsfaktor r= 0,210).

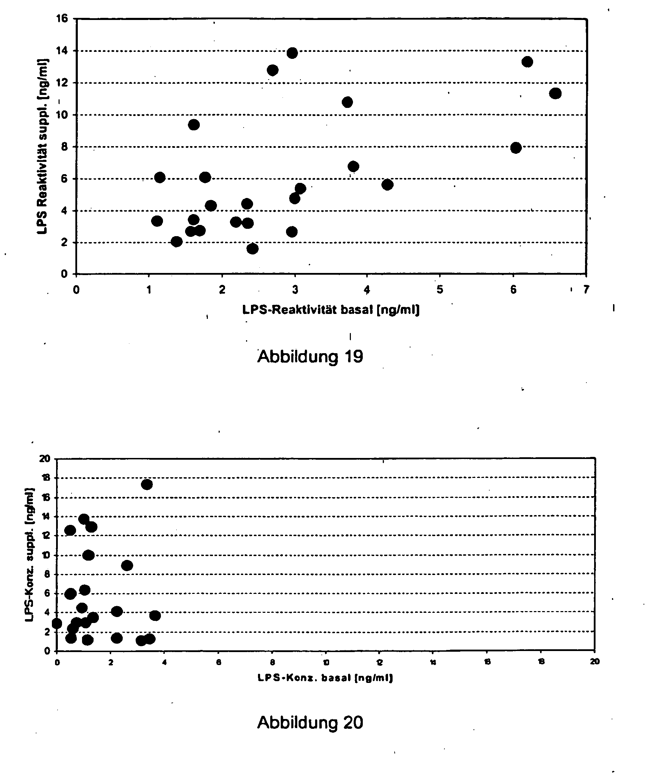

Abbildung 19 zeigt die Reaktivitäts-Ergebnisse weiterer Verfahren, bei denen 5 µl 0 oder 10 ng/ml LPS den Proben zugesetzt und im oxidativen Test mit dem Coamatic-Reagenz getestet wurden. Aus vorher 2,8 +- 1,6 ng/ml LPS wurden nach LPS Zusatz 6,2 ng/ml +- 3,8 ng/ml, der Korrelationsfaktor r = 0,574

Abbildung 20 zeigt die Ergebnisse weiterer Verfahren, bei denen Zusatz von 5 $\mu$l 10 ng/ml LPS bei Hitzebehandlung zu 5,8 +- 4,6 ng/ml LPS nach zuvor 1,5+- 1,1 ng/ml führt; Der Korrelationsfaktor ist r= 0,024. Dieser Korrelationsfaktor ist wesentlich kleiner als der durch Oxidation der Proben (r=0,574), d.h. es existiert eine extreme interindividuelle Streuung beim Supplementieren und Wiederfinden von LPS bei Testung mit der (stark denaturierenden) Hitzebehandlung im Gegensatz zur Oxidationsmethode.

Abbildung 21 zeigt die Reaktivitäts-Ergebnisse weiterer Verfahren, bei denen 10 $\mu$l NPG (gepoolt aus 12 Normalplasmen) supplementiert mit 0 - 10 ng/ml LPS mit 10 $\mu$l 60 mmol/l CT für 10 min 37˚C inkubiert wurden. Sodann wurden 10 $\mu$l 210 mmol/l Ascorbat, 210 mmol/l Arginin, pH 8,7, zugesetzt. Nach 3 min bei RT wurden 30 $\mu$l LAL-Reagenz (Coatest®, Chromogenix; zu rekonstituieren in 1,4 ml $H_2O$; ohne chromogenes Substrat) zugesetzt, und nach 0-90 min bei RT wurden 75 $\mu$l 750 mmol/l Arginin, pH 8,7, und 30 $\mu$l 2 mmol/l Ile-Glu-Gly-Arg-pNA zugesetzt und die lineare delta A bei 405 nm bestimmt. Aus Abbildung 21 ist zu erkennen, daß der optimale Linearisierungs-Zeitpunkt in diesem Ansatz 45 min RT ist (LPS-Eichgerade linear bis zu 10 ng/ml LPS). Abbildung 21 zeigt weiterhin, dass Limulus-Faktoren-Inkubationszeiten > 60 min RT sogar zu einem Abfall an LPS-Reaktivität führen ($\blacklozenge$,$\blacktriangle$). Dies ist bedingt durch eine Selbstzerstörung der Limulus-Faktoren, Anwesenheit von CS (wie im hyperbolic-rate-assay vor Zusatz des Linearisierungs-Reagenzes) schützt davor.

Abbildung 22 zeigt die Ergebnisse weiterer Verfahren, bei denen 10 $\mu$l NPG (aus 12 Normalplasmen) supplementiert mit 0-100 ng/ml LPS oder mit 0-100 $\mu$g/ml Zymosan A mit 10 ml 60 mmol/l CT für 10 min bei 37˚C inkubiert wurden. Sodann wurden 10$\mu$ l 210 mmol/l Arginin, 210 mmol/l Ascorbat (LPS-Assay) oder 2000 U/ml Polymyxin B (Glukane-Test, ZyA-Assay) und 30 $\mu$l LAL Reagenz Coamatic zugesetzt und delta A bestimmt. Aus Abbildung 22 ist zu erkennen, daß hier eine Linearität bis zu 2 $\mu$g/ml Zymosan A oder bis zu 100 ng/ml LPS auftritt. LPS Konzentrationen > 40 ng/ml konnten durch die eingesetzten 2000 U/ml Polymyxin B nicht mehr vollständig inhibiert werden.

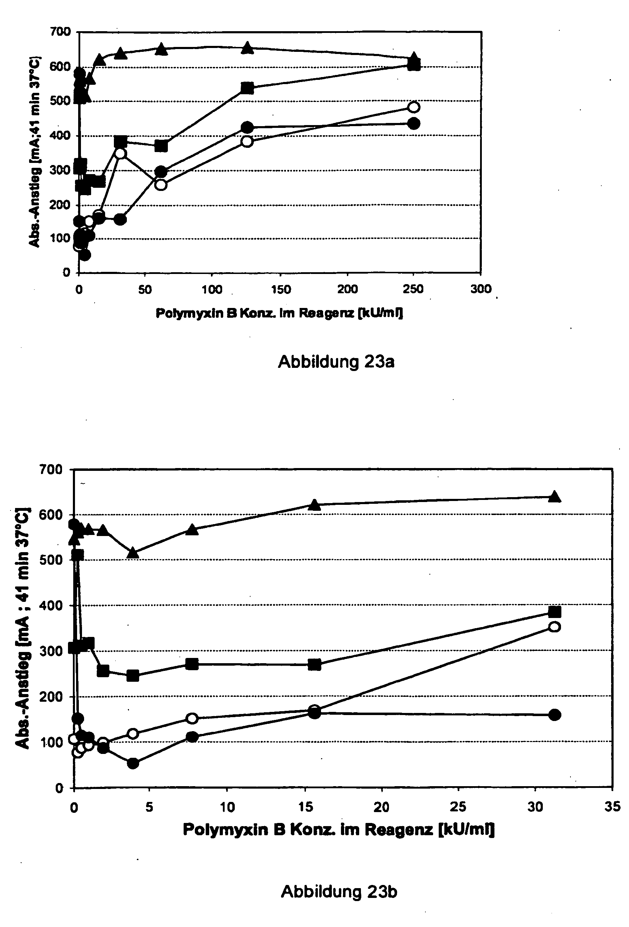

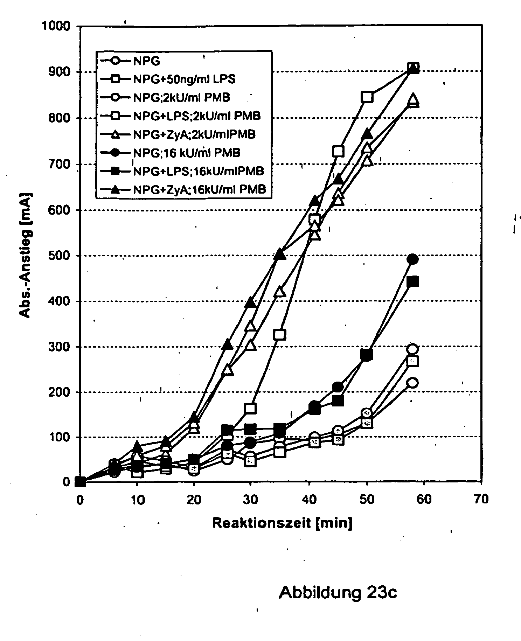

Abbildungen 23a-b zeigen die Ergebnisse weiterer Verfahren, bei denen 200 $\mu$l NPG ($\bigcirc$), NPG+50 ng/ml LPS ($\bullet$) oder +91 ng/ml LPS ($\blacksquare$), NPG+50 $\mu$g/ml ZyA ($\blacktriangle$) mit 200 $\mu$l 60 mmol/l CT in $H_2O$ für 10 min bei 37˚C inkubiert wurden. Je 20 $\mu$l davon wurden mit 10 $\mu$l 210 mmol/l Arginin, 210 mmol/l Ascorbat und 0 - 250 000 U/ml Polymyxin B bei 37˚C inkubiert und delta A bestimmt. Aus den Abbildungen 23a-c ist zu erkennen, daß Polymyxin B selbst das Limulus-System stimuliert. Abbildung 23c zeigt die Ergebnisse weiterer Verfahren, bei denen 200$\mu$l NPG ($\bigcirc$), +50 ng/ml LPS ($\square$), +2kU/ml PMB ($\bigcirc$ grau), +LPS und 2kU/ml PMB ($\square$ grau), +ZyA und 2kU/ml PMB ($\triangle$ grau), + 16kU/ml PMB ($\bullet$), + LPS und 16kU/ml PMB ($\blacksquare$), +ZyA und 16kU/ml PMB ($\blacktriangle$).

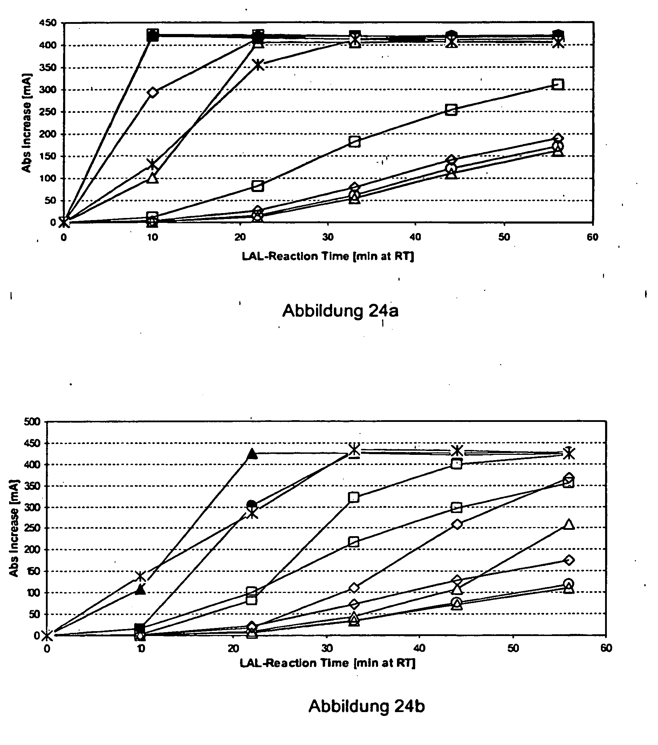

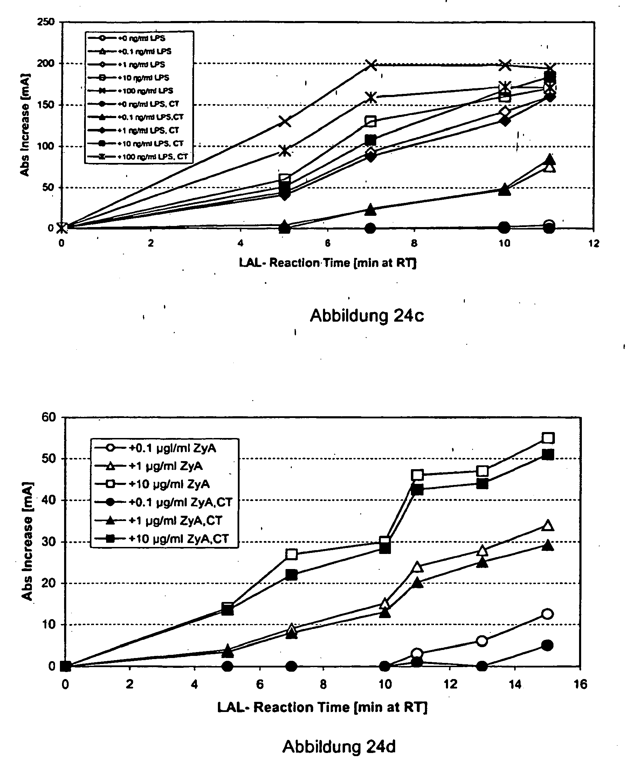

Abbildung 24 zeigt einen Vergleichsversuch des Verfahrens nach dem Stand der Technik mit 75˚C (15 min) Behandlung der Proben und dem erfindungsgemäßen Verfahren mit Oxidation der Proben mit 60 mM Chloramin-T® (10 min, 37˚C). Hierzu wurde 7.5 % Human-Albumin (Kabi, Stockholm, Schweden) basal enthaltend 0.5 $\mu$g/ml Zymosan A supplementiert mit 0 - 10000 ng/ml LPS (E. coli 0111:B4; gereinigt über Gel-Filtration; Product L 3012; Sigma, Deisenhofen, Germany) oder mit 0 - 100 $\mu$g/ml Zymosan A folgendermaßen untersucht:

A. Hitze-Behandlung

20 $\mu$l Probe wurde 10 fach verdünnt mit aqua dest. und 15 min bei RT (Abb. 24a) oder bei 75˚C (Wasserbad) (Abb. 24b) inkubiert. Dann wurde 20 $\mu$l Hitze-behandelte Probe in Doppelbestimmung mit 20 $\mu$l LAL-Reagenz (Pyrochrome®) bei RT inkubiert und die delta A bei 405 nm mittels eines Mikrotiterplattenphotometers (Milenia-DPC, Los Angeles, USA) bestimmt. Abb. 24a und b zeigen folgende Ansätze: + 0 ng/ml LPS ($\bigcirc$), + 0.1 ng/ml LPS (grau $\triangle$), + 1 ng/ml LPS (grau $\lozenge$), + 10 ng/ml LPS (grau $\square$), + 100 ng/ml LPS ($\bullet$), + 1000 ng/ml LPS ($\blacktriangle$), + 0.1 $\mu$g/ml ZyA ($\triangle$), + 1 $\mu$g/ml ZyA ($\lozenge$), + 10 $\mu$g/ml ZyA ($\square$), + 100 $\mu$g/ml ZyA (X). Man erkennt, dass + 1000 ng/ml LPS nach Hitzebehandlung wie + 0.1 ng/ml LPS bei RT (Abb.24a) agiert, d.h. 99.99 % des aktiven LPS wurden durch die Hitzebehandlung inaktiviert (Abb. 24b). Zymosan A erweist sich als Hitze-resistent.

B. Oxidations-Behandlung

5 $\mu$l Probe wurde in Doppelbestimmung mit 5 $\mu$l PBS oder 60 mM Chloramin-T® (CT) in PBS 10 min bei 37˚C (Wasserbad) inkubiert. Dann wurde 5 $\mu$l 230 mM Methionin in aqua dest. zugesetzt und nach 3 min RT wurde 15 $\mu$l LAL-Reagenz (Pyrochrome®) zugesetzt und die delta A bei 405 nm (RT) bestimmt. Man erkennt, dass sowohl die LPS-Reaktivität (Abb. 24c) als auch die Zymosan-Reaktivität Oxidations-resistent ist (Abb. 24d).

**Patentansprüche**

1. Verfahren zur Bestimmung des Gehaltes an aktivem und/oder freiem Lipopolysaccharid und/oder Lipid-A und/oder Glukan in biologischen Flüssigkeiten bei einem pH-Wert zwischen 6 und 10 umfassend

a) die Behandlung der Probe mit mindestens einem Oxidationsmittel aus der Gruppe der Singulett-Sauerstoff-Erzeuger,
b) den Zusatz von mindestens einem Limulus Amöbocyten - Faktor zur oxidierten Probe, und
c) die Bestimmung der Aktivierung mindestens eines der Limulus Amöbocyten -Faktoren.

2. Verfahren nach Anspruch 1, **dadurch gekennzeichnet, dass** als Oxidationsmittel ein Chloramin, insbesondere Chloramin-T® (N-Chlor-Toluol-Sulfonamid), eingesetzt wird.

3. Verfahren nach Anspruch 1, **dadurch gekennzeichnet, dass** Oxidationsmittel der Probe in einer Menge von 15-200 μmol, insbesondere 30-120 μmol, besonders bevorzugt 50-70 μmol pro ml Probenflüssigkeit zugesetzt wird.

4. Verfahren nach Anspruch 1, **dadurch gekennzeichnet, dass** nach der Durchführung der Oxidation die verbliebene Menge des Oxidans durch Zugabe eines Antioxidans inaktiviert wird, insbesondere durch Zugabe eines Quenchers für Singulett-Sauerstoff, vorzugsweise von Ascorbat oder Methionin.

5. Verfahren nach Anspruch 1, **dadurch gekennzeichnet, dass** die Bestimmung der Aktivierung mindestens eines der Faktoren des Limulus-Systems durch die Ermittlung der Menge des durch das Limulus-System erzeugten Chromophors einer durch aktivierten Limulus-Faktor spaltbaren chromogenen Verbindung erfolgt.

6. Verfahren nach Anspruch 1, **dadurch gekennzeichnet, dass** es sich bei der biologischen Flüssigkeit um Blut, Blutprodukte, Liquor, Urin, Saliva, Bronchoalveoläre Lavage, Aszites, Perikarderguß, Pleuraerguß, Milch, Gewebeflüssigkeiten, Lymphflüssigkeit und insbesondere Plasma handelt.

7. Verfahren nach Anspruch 1, **dadurch gekennzeichnet, dass** eine heparinhaltige biologische Flüssigkeit eingesetzt wird, der vor oder im Test mindestens ein Heparininhibitor zugesetzt worden ist.

8. Verfahren nach Anspruch 1, **dadurch gekennzeichnet, dass** eine biologische Flüssigkeit eingesetzt wird, die kein Heparin oder die bis zu 1 IU/ml Heparin enthält, und die Citrat oder EDTA enthält.

9. Verfahren nach Anspruch 1, **dadurch gekennzeichnet, dass** zur Linearisierung der Kinetik des Verfahrens vor oder während der Durchführung von Schritt c) eine Reagenz eingesetzt wird, die mindestens eine Guanidino-Verbindung oder ein Chaotropikum oder neben der Guanidino-Verbindung mindestens ein weiteres Chaotropikum enthält.

10. Verfahren nach Anspruch 9, **dadurch gekennzeichnet, dass** als Guanidino-Verbindung Arginin, und/oder als Chaotropikum ein Alkalimetallkation und/oder ein Erdalkalimetallkation eingesetzt wird.

11. Verfahren nach Anspruch 1, **dadurch gekennzeichnet, dass** dieses zwecks Differenzierung zwischen Reaktivität von LPS und Lipid-A und Glukan und Reaktivität von Glukan in Anwesenheit und in Abwesenheit eines LPS-Inhibitors durchgeführt wird und dass die in beiden Messreihen ermittelten Reaktivitäten voneinander subtrahiert werden, so dass auf diese Weise die durch Gukane und/oder durch LPS und/oder Lipid A hervorgerufene Reaktivität ermittelt wird.

12. Verfahren nach Anspruch 11, **dadurch gekennzeichnet, dass** als LPS-Inhibitor Polymyxin-B eingesetzt wird.

13. Verfahren nach Anspruch 1, **dadurch gekennzeichnet, dass** die biologische Flüssigkeit vor oder während Schritt a) mit (Human)Albumin verdünnt wird.

14. Verfahren nach Anspruch 1, **dadurch gekennzeichnet, dass** die biologische Flüssigkeit eine Proteinkonzentration von 30 -100 g/l, insbesondere von 60 - 90 g/l, aufweist.

15. Verfahren nach Anspruch 1, **dadurch gekennzeichnet, dass** der Probe zur Bestimmung der LPS- und/oder Lipid-A-Inhibitionskapazität eine definierte Menge an Lipopolysaccharid zugesetzt wird und der Gehalt an Lipopolysaccharid bestimmt wird.

16. Verfahren nach Anspruch 1, **dadurch gekennzeichnet, dass** der Probe zur Bestimmung der Glukan-Inhibitionskapazität eine definierte Menge an Glukan zugesetzt wird und der Gehalt an Glukan bestimmt wird.

**Claims**

1. Procedure to determine the content of active and/or free lipopolysaccharide and/or of lipid-A and/or of glucan in biologic liquids at a value of pH between 6 and 10, comprising

   a) the treatment of the sample with at least one oxidant out of the group of generators of singlet oxygen,
   b) the addition of at least one factor of amebocytes of Limulus to the oxidized sample, and
   c) the determination of the activation of at least one factor of amebocytes of Limulus.

2. Procedure according to claim 1, thereby **characterized**, that as oxidant a chloramine, particularly chloramine-T® (N-chlor-toluol-sulfonamide), is used.

3. Procedure according to claim 1, thereby **characterized**, that the oxidant is added to the sample in an amount of 15-200 μmoles, particularly 30-120 μmoles, especially preferred 50-70 μmoles per ml of liquid of sample.

4. Procedure according to claim 1, thereby **characterized**, that after the performance of the oxidation the remaining amount of oxidant is inactivated by addition of an antioxidant, particularly by addition of an inhibitor of singlet oxygen, preferably ascorbate or methionine.

5. Procedure according to claim 1, thereby **characterized**, that the activation of at least one factor of the system of Limulus is determined by measuring the amount of chromophore that is generated by the system of Limulus out of a chromogenic compound that can be degraded by an activated factor of Limulus.

6. Procedure according to claim 1, thereby **characterized**, that the biologic liquid is blood, products of blood, liquor, urine, saliva, bronchoalveolar lavage, ascites, pericardial or pleural effusions, milk, liquids of tissues, liquid of lymph, and particularly plasma.

7. Procedure according to claim 1, thereby **characterized**, that a biologic liquid that contains heparin is used that has been supplemented prior to the test or during the test with at least one inhibitor of heparin.

8. Procedure according to claim 1, thereby **characterized** that a biologic liquid is used that does not contain heparin or that contains up to 1 IU/ml heparin and that contains citrate or EDTA.

9. Procedure according to claim 1, thereby **characterized**, that the kinetic of the procedure is made linear prior to or during the performance of step c) with a reagent that contains at least one compound of guanidine and/or a chaotropic agent.

10. Procedure according to claim 9, thereby **characterized**, that as compound of guanidine arginine, and/or as chaotropic agent a cation of an alkali-metal and/or a cation of an earth-alkali-metal is used.

11. Procedure according to claim 1, thereby **characterized**, that this is performed to differentiate between reactivity of LPS and lipid-A and glucans and the reactivity of glucans in presence and in absence of an inhibitor of LPS and that the reactivities that have been determined in both approaches of test are subtracted from each other, to assess so the reactivity caused by LPS and/or lipid-A.

12. Procedure according to claim 11, thereby **characterized**, that as inhibitor of LPS polymyxin-B is used.

13. Procedure according to claim 1, thereby **characterized**, that the biologic liquid prior to or during step a) is diluted with (human) albumin.

14. Procedure according to claim 1, thereby **characterized**, that the biologic liquid has a concentration of protein of 30-100 g/l, particularly of 60 - 90 g/l.

15. Procedure according to claim 1, thereby **characterized** , that a defined amount of LPS and/or lipid-A is added to the sample and the content of lipopolysaccharide is determined to assess the capacity of inhibition of LPS and/or lipid-A.

16. Procedure according to claim 1, thereby **characterized**, that a defined amount of glucan is added to the sample and the content of glucan is determined to assess the capacity of inhibition of glucan.

**Revendications**

1. Procédure à déterminer le contenu de lipopolysaccharide et / ou de lipide-A et / ou de glucane actif et / ou libre dans liquides biologiques, a une valeur de pH entre 6 et 10, qui comprend

   a) traitement de l'échantillon avec au moins un oxydant sortent de la catégorie des générateurs d'oxygène singulet,
   b) l'ajout d'au moins un facteur d'amoebocytes de Limulus à l'échantillon oxydé, et
   c) la détermination de l'activation d'au moins un facteur d'amoebocytes de Limulus.

2. Procédure selon la revendication 1, **caractérisée par** là, que comme l'oxydant chloramine, et en particulier chloramine-T ® (N-chlore-toluol-sulfamide), est utilisé.

3. Procédure selon la revendication 1, **caractérisée par** là, que l'oxydant est ajouté à l'échantillon d'un montant de 15-200 $\mu$moles, en particulier 30-120 $\mu$moles, en particulier préféré 50-70 $\mu$moles par ml de liquide de l'échantillon.

4. Procédure selon la revendication 1, **caractérisée par** là, que après l'exécution de l'oxydation, la somme des oxydants est inactivé par addition d'un antioxydant, notamment par addition d'un inhibiteur de l'oxygène singulet, de préférence d'ascorbate ou de méthionine.

5. Procédure selon la revendication 1, **caractérisée par** là, que l'activation d'au moins un élément du système de Limulus est déterminé en mesurant la quantité de chromophore qui est générée par le système de Limulus sur un composé chromogénique qui peut être dégradé par un facteur activé de Limulus.

6. Procédure selon la revendication 1, **caractérisée par** là, que le liquide biologique est sang, produits du sang, liquor, urine, salive, lavage de broncho, ascite, épanchements pleuraux ou péricardiques, lait, liquides de tissus, liquide lymphatique, et en particulier plasma.

7. Procédure selon la revendication 1, **caractérisée par** là, qu'un liquide biologique qui contient l'héparine est utilisée qui a été complété avant l'essai ou pendant l'essai avec au moins un inhibiteur de l'héparine.

8. Procédure selon la revendication 1, **caractérisée** ainsi qu'un liquide biologique est utilisé qui ne contient pas d'héparine ou qui contient jusqu'à 1 UI / ml héparine et que contient citrate ou EDTA.

9. Procédure selon la revendication 1, **caractérisée par** là, que la cinétique de la procédure est fait linéaire avant ou pendant l'exécution de l'étape c), avec un réactif qui contient au moins un composé de guanidine et / ou d'un agent caotropique.

10. Procédure selon la revendication 9, **caractérisé par** là, que comme composé de guanidine arginine, et / ou comme un agent caotropique un cation d'un metal alcali et / ou un cation d'un metal alcali de terre est utilisée.

11. Procédure selon la revendication 1, **caractérisée par** là, que cela est fait pour différencier entre la réactivité de LPS et lipide-A et glucanes et la réactivité de glucanes en présence et en l'absence d'un inhibiteur de PS et que les réactivités qui ont été déterminées dans les deux méthodes de test sont soustraits les uns des autres, afin d'évaluer la réactivité causés par LPS et / ou lipide-A.

12. Procédure selon la revendication 11, **caractérisé par** là, que comme inhibiteur de LPS polymyxine-B est utilisé.

13. Procédure selon la revendication 1, **caractérisée par** là, que le liquide biologique avant ou pendant l'étape a) est dilué avec albumine (humain).

14. Procédure selon la revendication 1, **caractérisée par** là, que le liquide biologique contient une concentration de protéine de 30-100 g/l, en particulier de 60 - 90 g / l.

15. Procédure selon la revendication 1, **caractérisée par** là, qu'un montant défini de LPS et / ou de lipide-A est ajouté à l'échantillon et le contenu des lipopolysaccharide est déterminée à évaluer la capacité de l'inhibition de LPS et / ou de lipide-A.

16. Procédure selon la revendication 1, **caractérisée par** là, qu'un montant défini de glucane est ajouté à l'échantillon et le contenu de glucane est déterminée à évaluer la capacité de l'inhibition de glucane.

Abbildung 1

Abbildung 2

Abbildung 3

Abbildung 4

Abbildung 5

Abbildung 6

Abbildung 7

Abbildung 8

Abbildung 9

Abbildung 10

Abbildung 11

Abbildung 12

Abbildung 13

Abbildung14

Abbildung 15

Abbildung 16

Abbildung 17

Abbildung 18

27

Abbildung 19

Abbildung 20

Abbildung 21

Abbildung 22

Abbildung 23a

Abbildung 23b

Abbildung 23c

Abbildung 24a

Abbildung 24b

Abbildung 24c

Abbildung 24d

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3954663 A
- US 4495294 A
- DE 19549117
- EP 297597 A

**In der Beschreibung aufgeführte Nicht-Patentliteratur**

- **HURLEY J.C.** Endotoxemia: methods of detection and clinical correlates. *Clinical Microbiology Reviews,* 1995, vol. 8, 268-292
- **HURLEY J.** *Clin. Microbiol. Rev.,* 1995, vol. 8 (2), 268-292
- **OCHIAI et al.** *Microbiol. Immunol.,* 2002, vol. 46, 527-533
- **HURLEY J.C.** *Clinical Microbiology Reviews,* 1995, vol. 8, 272
- **OBAYASHI, T. et al.** *Clin. Chim. Acta,* 1985, vol. 149, 55-65
- *J. Lab. Clin Med.,* 1984, vol. 4, 321-330
- **TSUJI et al.** *Prog. Clin. Biol. Res.,* 1987, vol. 231, 443-457